(19) Europäisches Patentamt
European Patent Office
Office européen des brevets

(11) EP 3 812 461 A1

(12) # EUROPEAN PATENT APPLICATION
published in accordance with Art. 153(4) EPC

(43) Date of publication:
**28.04.2021 Bulletin 2021/17**

(21) Application number: **19821763.0**

(22) Date of filing: **28.05.2019**

(51) Int Cl.:
***C12N 15/11*** *(2006.01)* ***C12Q 1/6851*** *(2018.01)*
***C12Q 1/6886*** *(2018.01)* ***G01N 33/50*** *(2006.01)*

(86) International application number:
**PCT/JP2019/021098**

(87) International publication number:
**WO 2019/244575 (26.12.2019 Gazette 2019/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **21.06.2018 JP 2018118340**

(71) Applicant: **Public University Corporation Nagoya City University**
**Mizuho-ku**
**Nagoya-shi**
**Aichi 467-8601 (JP)**

(72) Inventors:
- **SHIMURA Takaya**
  **Nagoya-shi, Aichi 467-8601 (JP)**
- **IWASAKI Hiroyasu**
  **Nagoya-shi, Aichi 467-8601 (JP)**

(74) Representative: **Cabinet Becker et Associés**
**25, rue Louis le Grand**
**75002 Paris (FR)**

(54) **STOMACH CANCER BIOMARKER AND USE THEREOF**

(57) An object is to provide a highly practical, gastric cancer-specific, and minimally invasive biomarker. As gastric cancer biomarkers, hsa-miR-6807-5p, hsa-miR-6856-5p and hsa-miR-575 have been identified. The morbidity of gastric cancer is determined using expression levels of the microRNAs as an index.

EP 3 812 461 A1

## Description

[Technical Field]

The present invention relates to an index useful for testing/diagnosis of gastric cancer and a use thereof. Specifically, the present invention relates to a gastric cancer biomarker and a test method using the same. Priority is claimed on Japanese Patent Application No. 2018-118340, filed June 21, 2018, the entire content of which is incorporated herein by reference.

[Background Art]

The gold standard for gastric cancer diagnosis is an upper gastrointestinal endoscopy and a tissue diagnosis associated therewith, but is highly invasive and expensive. Therefore, the gold standard is not recommended for a screening test such as a screening for a healthy person. On the other hand, an upper gastrointestinal angiography has been widely introduced in the field of gastric cancer screening. However, it has not been scientifically proven that a gastric cancer mortality rate is reduced by such a test. In addition, in gastrointestinal angiography, a subject takes barium and then changes a position and a gastrointestinal lesion is picked up depending on the degree of deformation or expansion of the stomach wall due to barium storage and the state of barium attachment, and therefore it is difficult to identify early-stage cancer in which a tumor component remains on the surface of the stomach wall.

A serum tumor marker such as CEA or CA19-9 is used in blood tests, but the use of these as a diagnostic marker is not recommended due to the low sensitivity thereof (the sensitivity in diagnosing gastric cancer is reported to be 24.0% for CEA and 27.0% for CA19-9). In addition, although development of biomarkers specific to gastric cancer has progressed (for example, Patent Literatures 1 and 2), there are no examples of clinical application. Literatures relating to molecules forming a biomarker of the present invention are listed below (Patent Literatures 3 to 7 and Non-Patent Literature 1).

[Citation List]

[Patent Literature]

[Patent Literature 1]
Japanese Unexamined Patent Application, First Publication No. 2016-192909
[Patent Literature 2]
Pamphlet of PCT International Publication No. 2016/103761
[Patent Literature 3]
Pamphlet of PCT International Publication No. 2015/194627
[Patent Literature 4]
Pamphlet of PCT International Publication No. 2015/194615
[Patent Literature 5]
Pamphlet of PCT International Publication No. 2015/190586
[Patent Literature 6]
Pamphlet of PCT International Publication No. 2015/190542
[Patent Literature 7]
Pamphlet of PCT International Publication No. 2015/182781

[Non-Patent Literature]

[Non-Patent Literature 1]
https://doi.org/10.3892/mmr_00000199 (Title: MicroRNA profiling of human gastric cancer. Published in Molecular Medicine Reports, November 2009. DOI:10.3892/mmr_00000199)

[Summary of Invention]

[Technical Problem]

As described above, there is currently no biomarker useful for diagnosing gastric cancer. Therefore, an object of the present invention is to provide a highly practical, gastric cancer-specific, and minimally invasive biomarker.

[Solution to Problem]

The present inventors have conducted research with the aim of creating a biomarker specific to gastric cancer, with emphasis on a minimally invasive and simple test. Specifically, the present inventors attempted to identify a biomarker specific to gastric cancer from urine. As a result of detailed and comprehensive analysis using more than 300 samples, it was found that a specific microRNA enables determination of gastric cancer with high specificity and sensitivity. That is, the present inventors succeeded in identifying a biomarker having excellent diagnostic ability for gastric cancer. In addition, during the study, useful criteria for determining gastric cancer and useful information for clinical application were found. On the other hand, as a result of further study, it became clear that the identified microRNA functions as a gastric cancer biomarker not only in urine but also in serum, and various body fluids can be used as a sample for detecting the biomarker (that is, high versatility). In addition, as an important finding, it was found that the identified microRNAs are useful for the detection and diagnosis of early-stage (stage I) gastric cancer. On the other hand, it became clear that the identified microRNAs reflect the efficacy of a gastric cancer treatment very well. This fact indicates that the microRNA can also function as a useful biomarker not only for determining the efficacy of a gastric cancer treatment but also for "predicting gastric cancer recurrence" which is closely related to the efficacy.

Based on the above results and considerations, the following invention is provided.

[1] A gastric cancer biomarker, including:
one microRNA or a combination of two or more microRNAs selected from the group consisting of hsa-miR-6807-5p, hsa-miR-6856-5p, and hsa-miR-575.

[2] The gastric cancer biomarker according to [1], in which the gastric cancer biomarker is detected in a urine sample or a blood sample.

[3] A test method for gastric cancer, in which an expression level of the gastric cancer biomarker according to [1] or [2] is used as an index.

[4] The gastric cancer biomarker according to [1] or [2], including: hsa-miR-6807-5p or hsa-miR-6856-5p; or
a combination of hsa-miR-6807-5p and hsa-miR-6856-5p, in which the gastric cancer biomarker is used for detecting early gastric cancer.

[5] A test method for early gastric cancer, in which an expression level of the gastric cancer biomarker according to [4] is used as an index.

[6] The gastric cancer biomarker according to [1] or [2], including: hsa-miR-6807-5p or hsa-miR-6856-5p; or
a combination of hsa-miR-6807-5p and hsa-miR-6856-5p, in which the gastric cancer biomarker is used for determining the efficacy of a gastric cancer treatment or predicting gastric cancer recurrence.

[7] The method according to [3], including the steps of (1) to (3) below:

(1) a step of detecting the gastric cancer biomarker in a sample collected from a subject and determining an expression level;
(2) a step of obtaining a result of a Helicobacter pylori infection test of the subject; and
(3) a step of determining morbidity of gastric cancer, based on the expression level determined in the step (1) and the result of the Helicobacter pylori infection test prepared in the step (2).

[8] The method according to [7], in which the gastric cancer biomarker to be detected in the step (1) is hsa-miR-6807-5p and hsa-miR-6856-5p, and the expression level of the gastric cancer biomarker is determined by normalization using hsa-miR-3610 and hsa-miR-4669 as normalization factors.

[9] The method according to [7], in which the gastric cancer biomarker to be detected in the step (1) is hsa-miR-575, and the expression level of the gastric cancer biomarker is determined by normalization using hsa-miR-3610, hsa-miR-4669, and hsa-miR-6803-5p as normalization factors.

[10] The method according to any one of [7] to [9], in which the level of the gastric cancer biomarker has a positive correlation with the morbidity of gastric cancer.

[11] The method according to any one of [7] to [10], in which the gastric cancer biomarker is detected by a qRT-PCR method.

[12] The method according to [11],
in which, after obtaining a value (ΔCt value) obtained by subtracting a Ct value of a normalization factor from a Ct value of the gastric cancer biomarker, the morbidity of gastric cancer is determined using two mathematical expressions with the ΔCt value as a variable, the two mathematical expressions being set separately for a case where the subject is positive for Helicobacter pylori infection and a case where the subject is negative for Helicobacter pylori infection.

[13] The method according to [5], including the steps of (1) to (3) below:

(1) a step of detecting the gastric cancer biomarker in a sample collected from a subject and determining an expression level;
(2) a step of obtaining a result of a Helicobacter pylori infection test of the subject; and
(3) a step of determining morbidity of early gastric cancer, based on the expression level determined in the step (1) and the result of the Helicobacter pylori infection test prepared in the step (2).

[14] The method according to [13], in which the gastric cancer biomarker to be detected in the step (1) is hsa-miR-6807-5p and hsa-miR-6856-5p, and the expression level of the gastric cancer biomarker is determined by normalization using hsa-miR-3610 and hsa-miR-4669 as normalization factors.
[15] The method according to [13] or [14], in which the level of the gastric cancer biomarker has a positive correlation with a stage of gastric cancer.
[16] The method according to any one of [13] to [15], in which the gastric cancer biomarker is detected by a qRT-PCR method.
[17] The method according to [16],
in which, after obtaining a value (ΔCt value) obtained by subtracting a Ct value of a normalization factor from a Ct value of the gastric cancer biomarker, the morbidity of early gastric cancer is determined using two mathematical expressions with the ΔCt value as a variable, the two mathematical expressions being set separately for a case where the subject is positive for Helicobacter pylori infection and a case where the subject is negative for Helicobacter pylori infection.
[18] The method according to any one of [7] to [17], in which the sample is urine or blood.
[19] A gastric cancer test kit, including: a reagent for detecting the gastric cancer biomarker according to [1]; and an instruction manual.
[20] A gastric cancer test kit, including: a reagent for detecting the gastric cancer biomarker according to [4] or [6]; and an instruction manual.
[21] The gastric cancer test kit according to [19] or [20], in which the reagent is a combination of a reagent for detecting hsa-miR-6807-5p and a reagent for detecting hsa-miR-6856-5p.
[22] The gastric cancer test kit according to [19], in which the reagent is a reagent for detecting hsa-miR-575.
[23] The gastric cancer test kit according to any one of [19] to [22], further including: one or more elements selected from the group consisting of a reagent for extracting a microRNA, a normalization factor-specific primer, a DNA polymerase, a reverse transcriptase, dNTPs, a urine sample collection container, a reactor, and a detector.

[Brief Description of Drawings]

Fig. 1 shows a research and analysis scheme.
Fig. 2 shows a breakdown of an analysis cohort.
Fig. 3 shows results of a comprehensive analysis (miRNA array).
Fig. 4 shows results of a comprehensive analysis (miRNA array).
Fig. 5 shows results of a training set analysis (normalization with miR-3610 + miR-4669). 100 times $2^{-\Delta Ct}$ was used to calculate an odds ratio.
Fig. 6 shows an ROC curve of a training set (urinary miRNA diagnostic panel).
Fig. 7 shows results of a validation set analysis (normalization with miR-3610 + miR-4669). 100 times $2^{-\Delta Ct}$ was used to calculate an odds ratio.
Fig. 8 shows an ROC curve of a validation set (urinary miRNA diagnostic panel).
Fig. 9 shows a risk determination for gastric cancer using a model expression ($-1.471 + 1.7486 \times (HP) + 63.424 \times 2^{-\Delta Ct(miR\text{-}6807\text{-}5p)} + 7.8641 \times 2^{-\Delta Ct(miR\text{-}6856\text{-}5p)}$) (however, in the expression, HP is 1 for a case of positive for Helicobacter pylori infection, and HP is 0 for a case of negative for Helicobacter pylori infection). A gastric cancer prevalence was calculated as 0.2%.
Fig. 10 shows results of a training set analysis (normalization with miR-3610 + miR-4669 + miR-6803-5p). 100 times $2^{-\Delta Ct}$ was used to calculate an odds ratio.
Fig. 11 shows an ROC curve of a training set (urinary miRNA diagnostic panel).
Fig. 12 shows results of a validation set analysis (normalization with miR-3610 + miR-4669 + miR-6803-5p). 100 times $2^{-\Delta Ct}$ was used to calculate an odds ratio.
Fig. 13 shows an ROC curve of a validation set (urinary miRNA diagnostic panel).
Fig. 14 shows a risk determination for gastric cancer using a model expression ($-1.2062 + 2.2727 \times (HP) + 18.4419$

$\times 2^{-\Delta Ct(miR-575)}$) (however, in the expression, HP is 1 for a case of positive for Helicobacter pylori infection, and HP is 0 for a case of negative for Helicobacter pylori infection). A gastric cancer prevalence was calculated as 0.2%.

Fig. 15 shows results of a verification experiment using a serum miRNA as a biomarker. Expressions of biomarkers were compared between healthy people and gastric cancer patients, with normalization with miR-3610 + miR-4669.

Fig. 16 shows results of a verification experiment using a serum miRNA as a biomarker. Fig. 16 shows an ROC curve (serum miRNA diagnostic panel) in a case of normalization with miR-3610 + miR-4669.

Fig. 17 shows results of a verification experiment using a serum miRNA as a biomarker. Expressions of biomarkers were compared between healthy people and gastric cancer patients, with normalization with miR-3610 + miR-4669 + miR-6803-5p.

Fig. 18 shows results of a verification experiment using a serum miRNA as a biomarker. Fig. 18 shows an ROC curve (serum miRNA diagnostic panel) in a case of normalization with miR-3610 + miR-4669 + miR-6803-5p.

Fig. 19 shows results of a comparative study between healthy people (149 cases) and early (stage I) gastric cancer cases (95 cases).

Fig. 20 shows ROC curves of healthy people (149 cases) and early (stage I) gastric cancer cases (95 cases).

Fig. 21 shows a correlation among a miR-6807-5p expression level (left) and a miR-6856-5p expression level (right) and a clinical stage.

Fig. 22 shows expression levels of miR-6807-5p and miR-6856-5p miRNA in gastric cancer tissue.

Fig. 23 shows changes in a urinary miRNA before and after gastric cancer resection.

[Description of Embodiments]

1. Gastric cancer biomarker

A first aspect of the present invention relates to a gastric cancer biomarker. In the present specification, the term "gastric cancer biomarker" refers to a biomolecule that is a morbidity index of gastric cancer. The biomarker of the present invention is useful for detecting gastric cancer (understanding that a patient has gastric cancer), particularly for detecting early gastric cancer. In addition, the biomarker can be used for determining the efficacy of a gastric cancer treatment and predicting a recurrence of gastric cancer. That is, the biomarker can also be used for evaluating a therapeutic effect.

Gastric cancer is a general term for epithelial malignant tumors that occur in the stomach, and is subdivided according to a histological type, a stage, and the like. In early-stage gastric cancer, there are few subjective symptoms, and even if the gastric cancer progresses considerably, it manifests as asymptomatic in some cases. Therefore, regular gastric cancer screening is recommended to detect gastric cancer at an early stage. For a diagnosis of gastric cancer, a blood test, a gastric X-ray test, an endoscopy, a pathological test, an ultrasonography, a CT test, an enema test, and the like are performed.

In the present specification, the term "biomolecule" refers to a molecule (compound) found in a living body. In the present invention, a microRNA, which is a biomolecule, is used as the biomarker, but when using the microRNA (typically applying to a test method), the molecule in a sample separated from the living body is used. Human body fluids such as urine, blood (serum, plasma, or whole blood), saliva, sweat, and digestive juice are used as the sample, but urine or blood is preferably used as the sample. That is, in a preferred aspect, a microRNA in urine or blood is the biomarker of the present invention.

The microRNA is a small RNA of about 22 bases existing in the living body. The microRNA suppresses protein production by binding to a target gene that has a partially complementary sequence in a 3' untranslated region (3'UTR) and suppressing mRNA destabilization and translation. This function of the microRNA is a part of an important mechanism for post-transcriptional expression regulation of genes.

The biomarker of the present invention includes a specific microRNA, that is, hsa-miR-6807-5p or hsa-miR-6856-5p, or hsa-miR-575 or a combination of two or more thereof. The combination is not particularly limited, but a combination of hsa-miR-6807-5p and hsa-miR-6856-5p is preferably adopted. In a case where hsa-miR-6807-5p or hsa-miR-6856-5p, or the combination of hsa-miR-6807-5p and hsa-miR-6856-5p is adopted, the biomarker is particularly useful for detecting early gastric cancer. Stages of the gastric cancer are broadly divided into stages I, II, III, and IV. Unless otherwise specified, the "early gastric cancer" in the present invention is stage I gastric cancer. Also in a case where the biomarker of the present invention is used for determining the efficacy of a gastric cancer treatment or predicting gastric cancer recurrence, hsa-miR-6807-5p or hsa-miR-6856-5p, or a combination of hsa-miR-6807-5p and hsa-miR-6856-5p is adopted.

hsa-miR-6807-5p is a microRNA consisting of the sequence GUGAGCCAGUGGAAUGGAGAGG (SEQ ID NO: 11). Similarly, hsa-miR-6856-5p is a microRNA consisting of the sequence AAGAGAGGAGCAGUGGUGCUGUGG (SEQ ID NO: 12) and hsa-miR-575 is a microRNA consisting of the sequence GAGCCAGUUGGACAGGAGC (SEQ ID NO: 1).

For convenience of explanation, in the present specification, "hsa-" may be omitted from names of the micro-RNAs in the present specification.

2. Test using gastric cancer biomarker

A second aspect of the present invention provides a test method for gastric cancer (hereinafter, also referred to as a "test method of the present invention") with respect to a use of the biomarker of the present invention. The test method of the present invention is useful as means for simply detecting gastric cancer. According to the test method of the present invention, an objective basis is provided that enables determination of whether a person has gastric cancer (presence or absence of morbidity). In addition, one aspect of the test method of the present invention is useful for detecting early gastric cancer, and in a case of this aspect, an objective basis useful for determining whether a person has early gastric cancer is obtained. Information (test result) provided by the test method of the present invention is based on an objective index called a biomarker (biomolecule) that reflects a state or change in the living body, and enables the determination of gastric cancer by itself. However, the information may be used as a supplement to make a final determination (typically a definitive diagnosis) in consideration of other indices as necessary. In addition, in order to clarify a distinction from a diagnosis including determination of a doctor, the test method of the present invention can also be referred to as a "method for assisting a gastric cancer test". The present invention provides useful information for the diagnosis of gastric cancer by using an objective (index) regardless of the determination of a doctor.

In the test method of the present invention, the expression level of the biomarker of the present invention in a sample derived from a subject is used as an index. A "level" here typically means "amount" or "concentration". However, the term "level" is also used for a case of indicating whether a molecule to be detected can be detected (that is, presence or absence of apparent existence) in accordance with conventions and technical common sense.

Typically, the test method of the present invention performs the following the steps of (1) to (3).

(1) A step of detecting the gastric cancer biomarker in a sample collected from a subject and determining an expression level

(2) A step of obtaining a result of a Helicobacter pylori infection test of the subj ect

(3) A step of determining morbidity of gastric cancer, based on the expression level determined in the step (1) and the result of the Helicobacter pylori infection test prepared in the step (2)

Step (1)

In the step (1), a sample collected from a subject is prepared, the biomarker of the present invention (gastric cancer biomarker) is detected, and an expression level is determined. In a case of having a purpose of detecting early gastric cancer, hsa-miR-6807-5p or hsa-miR-6856-5p, or the combination of hsa-miR-6807-5p and hsa-miR-6856-5p is adopted as the biomarkers. The subject is not particularly limited. That is, the present invention can be widely applied to a person who needs to determine the presence or absence of morbidity of gastric cancer. For example, in addition to those who may have or are suspected of having gastric cancer, such as those who have signs of gastric cancer or those who have symptoms characteristic of gastric cancer, those who have no subjective symptoms (including healthy people) can be a subject.

The sample is collected prior to the implementation of the present invention. Various body fluids such as urine, blood (serum, plasma, or whole blood), saliva, sweat, and digestive juice are used as the sample. In a preferred aspect, urine, serum, or plasma is used as the sample. The sample may be collected and prepared by a conventional method.

In this step, the biomarker in the sample is detected, but it is not essential to strictly quantify the expression level of the biomarker. That is, it suffices as long as the biomarker can be detected to the extent that the morbidity of gastric cancer can be determined in the subsequent step (3). For example, detection can be performed so that it can be determined whether the level of the biomarker in the sample exceeds a predetermined reference value.

A method for detecting the biomarker is not particularly limited. For example, the biomarker can be detected by a microarray, which is a method using a nucleic acid amplification reaction represented by a qRT-PCR method. As the nucleic acid amplification reaction, a polymerase chain reaction (PCR) method or a modified method thereof, as well as a Loop-Mediated Isothermal Amplification (LAMP) method (Tsugunori Notomi et al. Nucleic Acids Research, Vol. 28, No. 12, e63, 2000; Kentaro Nagamine, Keiko Watanabe et al. Clinical Chemistry, Vol. 47, No. 9, 1742-1743, 2001), an Isothermal and Chimeric primer-initiated Amplification of Nucleic acids (ICAN) method (Japanese Patent No. 3433929, Japanese Patent No. 3883476), a Nucleic Acid Sequence-Based Amplification (NASBA) method, a Ligase Chain Reaction (LCR) method, a Self-sustained Sequence Replication (3SR) method, a Standard Displacement Amplification (SDA) method, a Transcription-Mediated Amplification (TMA) method, and a Rolling Circle Amplification (RCA) can be adopted.

The expression level of the biomarker is determined from the detection results. When determining the expression level, normalization is usually performed in order to improve accuracy, objectivity, and the like. For the normalization, the detection results of the microRNAs with constitutive expression are used. In a case where the combination of hsa-miR-6807-5p and hsa-miR-6856-5p is used as the biomarker, hsa-miR-3610 and hsa-miR-4669 are preferably used as normalization factors. That is, the detection results of hsa-miR-6807-5p and hsa-miR-6856-5p are corrected using the detection results of hsa-miR-3610 and hsa-miR-4669 to obtain the expression level used for the determination in the step (3). Similarly, in a case where hsa-miR-575 is used as the biomarker, hsa-miR-3610, hsa-miR-4669, and hsa-miR-6803-5p are preferably used as the normalization factors to determine a normalized expression level.

As long as the biomarker is measured by the qRT-PCR method, a Ct value (Threshold Cycle) may be used to determine the expression level. In this case, for example, a value (ΔCt value) obtained by subtracting a Ct value of a normalization factor from a Ct value of the biomarker is obtained, and the ΔCt value is used as the expression level used for the determination in the step (3).

Step (2)

In the step (2), a result of a Helicobacter pylori infection test of the subject is prepared. The Helicobacter pylori infection test is performed prior to the implementation of the present invention. A type, a method, or the like of the Helicobacter pylori infection test are not particularly limited, and for example, a urea breath test, a fecal antigen test, a blood or urine anti-Helicobacter pylori IgG antibody test, an endoscopy test, a histopathological test, a rapid urease test, and a culture method may be used for the test. The order of the step (2) and the step (1) above does not matter. That is, any of the steps may be performed first, or may be performed in parallel.

Step (3)

In the step (3), the morbidity of gastric cancer is determined, based on the expression level determined in the step (1) and the result of the Helicobacter pylori infection test prepared in the step (2). In a case of having a purpose of detecting early gastric cancer, the morbidity of early gastric cancer is determined. In order to enable accurate determination, for example, the determination may be performed by comparing the expression level obtained in the step (1) with the expression level of a control (control sample) or in light of criteria set based on the expression level of the control. As the control, for example, a biomarker expression level of healthy people and/or a biomarker expression level of gastric cancer patients, or a biomarker expression level of early (stage I) gastric cancer patients (in a case of having the purpose to detect early gastric cancer) can be used.

As shown in examples described later, hsa-miR-6807-5p, hsa-miR-6856-5p, and hsa-miR-575 serving as the biomarkers of the present invention all showed a significant increase in expression in gastric cancer cases. That is, the expression level of the biomarker of the present invention shows a positive correlation with the morbidity of gastric cancer. Therefore, regarding the expression level of the biomarker, a high level is a basic index (determination criteria) for morbidity of gastric cancer. On the other hand, hsa-miR-6807-5p and hsa-miR-6856-5p showed a significant increase in expression in early (stage I) gastric cancer cases. Therefore, a high expression level of the biomarkers is an index (determination criteria) for morbidity of early gastric cancer.

The determination in the step (3) may be qualitative, semi-quantitative, or quantitative. Examples of the qualitative determination and the quantitative determination are shown below. The following examples are examples of a case of determining the morbidity of gastric cancer, but the same applies to the determination of the morbidity of early gastric cancer. The number of determination classes, the expression level of the biomarker associated with each determination class, the determination result, and the like can be freely set through preliminary experiments and the like without being bound by the following examples. Further, a reference value or a cutoff value used for the determination may be set while considering, for example, the sample to be used and the required accuracy (reliability). When setting the reference value and the cutoff value, statistical analysis using a large number of samples may be used. In a case of using a combination of two microRNAs (for example, the combination of hsa-miR-6807-5p and hsa-miR-6856-5p) is used as the biomarker, the reference value and the cutoff value are set for each microRNA. Alternatively, a discriminant for the combination may be created and the reference value or the cutoff value for the discriminant may be set. As is clear from the determination criteria, the determination here can be performed automatically or mechanically without the determination of a person having specialized knowledge such as a doctor or a laboratory technician.

(Example of qualitative determination)

When Helicobacter pylori infection is positive and the biomarker expression level is higher than a reference value A, or when Helicobacter pylori infection is negative and the biomarker expression level is higher than a reference value B, it is determined that "a person has gastric cancer". When Helicobacter pylori infection is positive and the biomarker expression level is equal to or lower than the reference value A, or when the Helicobacter pylori infection is negative and the biomarker expression level is equal to or lower than the reference value B, it is determined that "a person does not have gastric cancer".

(Example of quantitative determination)

As shown below, a possibility (%) of morbidity is preset for each range of expression levels of cases of positive for Helicobacter pylori infection and negative for Helicobacter pylori infection, and the possibility (%) of morbidity is determined.

(i) Positive for Helicobacter pylori infection

a < Expression level: Possibility of having gastric cancer of higher than 80%
b < Expression level ≤ a: Possibility of having gastric cancer of 20% to 80%
Expression level < b: Possibility of having gastric cancer of lower than 20%

(ii) negative for Helicobacter pylori infection

c < Expression level: Possibility of having gastric cancer of higher than 20%
d < Expression level ≤ c: Possibility of having gastric cancer of 5% to 20%
Expression level < d: Possibility of having gastric cancer of lower than 5%

As described above, when the qRT-PCR method is used for the detection of the biomarker in the step (1), the determination of the step (3) is preferably performed by using the value (ΔCt value) obtained by subtracting a Ct value of a normalization factor from a Ct value of the biomarker as the biomarker expression level. At the time of determination, two mathematical expressions with the ΔCt value as a variable, the two mathematical expressions being set separately for a case where the subject is positive for Helicobacter pylori infection and a case where the subject is negative for Helicobacter pylori infection may be used. For example, when the combination of hsa-miR-6807-5p and hsa-miR-6856-5p is used as the biomarker, two mathematical expressions (Mathematical expression 1 in a case of being positive for Helicobacter pylori infection and Mathematical expression 2 in a case of being negative for Helicobacter pylori infection) with a ΔCt value of hsa-miR-6807-5p (denoted as ΔCt (miR-6807-5p)) and a ΔCt value of hsa-miR-6856-5p (denoted as ΔCt (miR-6856-5p)) as variables are set, and when a calculated value (which is calculated by Mathematical expression 1 when the subject is positive for Helicobacter pylori infection and is calculated by Mathematical expression 2 when the subject is negative for Helicobacter pylori infection) is equal to or greater than the reference value, it is determined that "a person has gastric cancer" or "a person is highly likely to have gastric cancer" and when the calculated value is smaller than the reference value, it is determined that "a person does not have gastric cancer" or "a person is less likely to have gastric cancer" (referred to as "Determination example 1"). On the other hand, when one of hsa-miR-6807-5p, hsa-miR-6856-5p, and hsa-miR-575 is used as the biomarker, a condition including two mathematical expressions (Mathematical expression 3 in a case of being positive for Helicobacter pylori infection and Mathematical expression 4 in a case of being negative for Helicobacter pylori infection) with the ΔCt value as variable is used, and when the condition is satisfied it is determined that "a person has gastric cancer" or "a person is highly likely to have gastric cancer", and the condition is not satisfied, it is determined that "a person does not have gastric cancer" or "it is less likely to have gastric cancer" (referred to as "Determination example 2"). Hereinafter, examples of the mathematical expressions used in Determination example 1 and Determination example 2 will be shown. Determination example 2 is particularly effective when hsa-miR-575 is used as the biomarker.

<Determination example 1>

In a case of being positive for Helicobacter pylori infection

$$\text{(Mathematical expression 1): } a \times 2^{-\Delta Ct(\text{miR-6807-5p})} + b \times 2^{-\Delta Ct(\text{miR-6856-5p})}$$

In a case of being negative for Helicobacter pylori infection

$$\text{(Mathematical expression 2): } c \times 2^{-\Delta Ct(\text{miR-6807-5p})} + d \times 2^{-\Delta Ct(\text{miR-6856-5p})}$$

However, a to d in the expression are constants. Specific examples of the constants a to d are a = 65, b = 14, c = 63, and d = 5 (see examples described below for details).

<Determination example 2>

In a case of being positive for Helicobacter pylori infection

$$\text{(Mathematical expression 3)}: 2^{-\Delta Ct} > e$$

In a case of being negative for Helicobacter pylori infection

$$\text{(Mathematical expression 4)}: 2^{-\Delta Ct} > f$$

However, e and f in the expression are constants. Specific examples of the constants e and f are e = 0.03446916 and f = 0.02673549 (see examples described below for details).

The determination result in the step (3) becomes useful information for the diagnosis of gastric cancer, and is useful not only for diagnosis of gastric cancer, but also for early detection of gastric cancer and determination of more appropriate treatment policy (such as selection of an effective treatment method). Also, the determination result can provide an opportunity to undergo a detailed test (for example, a secondary screening).

Further, as described above, the biomarker of the present invention can also be used for determining the efficacy of a gastric cancer treatment and predicting a recurrence of gastric cancer. In a case of the uses, for example, a step (i) of detecting the biomarker in a sample collected from a subject and determining the expression level is performed and then the efficacy of a gastric cancer treatment or possibility (risk) of recurrence of gastric cancer is determined based on the expression level determined in the step (i) (step (ii)). The step (i) is the same as the step (1) above. However, a person who needs to determine the efficacy of a gastric cancer treatment or recurrence of gastric cancer, that is, a person who has gastric cancer and has been treated (treatment-experienced person) is a subject. The treatment here is not particularly limited as long as the therapeutic effect as a curative therapy can be expected, but typically corresponds to a surgical treatment (usually, endoscopic tumor resection, laparoscopic surgery, reduction surgery, routine surgery or extended surgery are selected in consideration of a stage or a pathological condition, and also, lymph node dissection is performed as needed). In the determination of the step (ii), based on a fact that biomarkers (hsa-miR-6807-5p and hsa-miR-6856-5p) were significantly and remarkably reduced in expression in all cases after gastric cancer resection, a low expression level of the biomarker is an index (determination criterion) of high efficacy for a gastric cancer treatment (in a case of determining the efficacy of a gastric cancer treatment) and low possibility (risk) of gastric cancer recurrence (in a case of predicting a recurrence of gastric cancer). The determination may be qualitative, semi-quantitative, or quantitative, and the reference value, cutoff value, or the like used for the determination are in accordance with the step (3) above.

3. Gastric cancer test kit

The present invention also provides a gastric cancer test kit. The kit of the present invention contains a reagent for detecting the biomarker of the present invention (a reagent for detecting the biomarker) as an essential element. An example of the reagent for detecting the biomarker is a biomarker-specific primer.

In a preferred aspect, the kit includes, as the reagent for detecting the biomarker, a combination of a reagent for detecting hsa-miR-6807-5p (for example, an hsa-miR-6807-5p-specific primer) and a reagent for detecting hsa-miR-6856-5p (for example, an hsa-miR-6856-5p-specific primer). In this aspect, it is preferable that the kit contain the reagents for detecting hsa-miR-3610 and hsa-miR-4669 as the normalization factors, that is, a reagent for detecting hsa-miR-3610 (for example, an hsa-miR-3610-specific primer) and a reagent for detecting hsa-miR-4669 (for example, an hsa-miR-4669-specific primer).

In another preferred aspect, the kit contains a reagent for detecting hsa-miR-575 (for example, an hsa-miR-575-specific primer). In this aspect, it is preferable that the kit contain the reagents for detecting hsa-miR-3610, hsa-miR-4669, and hsa-miR-6803-5p as the normalization factors, that is, the reagent for detecting hsa-miR-3610 (for example, the hsa-miR-3610-specific primer), the reagent for detecting hsa-miR-4669 (for example, the hsa-miR-4669-specific primer), and a reagent for detecting hsa-miR-6803-5p (for example, an hsa-miR-6803-5p-specific primer).

The kit of the present invention usually includes an instruction manual. The kit may include other reagents (such as a reagent for extracting a microRNA, a DNA polymerase, and a reverse transcriptase, and dNTPs), and devices or instruments (such as a urine sample collection container, a reactor, and a detector) which are used when implementing the gastric cancer test method.

[Examples]

The following studies were conducted to find a gastric cancer-specific biomarker (a gastric cancer biomarker).

1. Method

(1) Experimental scheme/target/clinical sample

First, from all 372 cases in a cohort (healthy people: 197 cases, gastric cancer patients: 175 cases), 306 cases with randomly matched age and gender were selected and randomly classified into three groups of: (i) 8 cases in a comprehensive analysis cohort (healthy people and gastric cancer patients: 4 cases of each); (ii) 190 cases in a training set (healthy people and gastric cancer patients: 95 cases of each); and (iii) 108 cases in a validation set (healthy people/gastric cancer patients: 54 cases of each) (Figs. 1 and 2).

First, a miRNA array analysis was performed using the 8 cases of the comprehensive analysis cohort of (i), and miRNAs that were significantly increased or decreased in the urine of gastric cancer cases were extracted. Next, in the 190 cases of the training set of (ii), each miRNA extracted in (i) was measured by the qPCR method, significant gastric cancer biomarkers were extracted by a multivariate analysis, and a diagnostic panel was constructed. Finally, using the independent 108 cases of the validation set of (iii), accuracy of the diagnostic panel established in (ii) was verified.

(2) RNA extraction/cDNA synthesis

Immediately after collection, miRNA was extracted from 200 μl of urine sample cryopreserved at -80°C using miRNeasy Serum/Plasma Kit (Qiagen, Valencia, CA) according to a protocol. Using the extracted miRNA as a template, cDNA synthesis was performed using the TaqMan Advanced MicroRNA cDNA Synthesis Kit (ThermoFisher Scientific, Waltham, USA) according to the protocol.

(3) Microarray analysis

Using the extracted miRNA, analysis was performed by SurePrint miRNA microarray (Agilent, Santa Clara, USA). GeneSpringGX (Agilent) was used for data analysis.

(4) qPCR

The qRT-PCR was performed using a 7500 Fast real-time PCR system (ThermoFisher Scientific, Watham, USA) using a TaqMan Advanced MicroRNA Assay using the synthesized cDNA. A thermal cycle and sequences of the primers used are listed in tables below (Tables 1 and 2).

qRT-PCR thermal cycle

[Table 1]

| Step | Temperature | Time | Number of cycles |
|---|---|---|---|
| Oxygen activation | 95°C | 20 sec | 1 |
| Denaturation | 95°C | 3 sec | 40 |
| Annealing/Extension | 60°C | 30 sec | |

TaqMan Advanced MicroRNA Assays Used

[Table 2]

| (Target microRNA) | Assay ID | Context Sequence |
|---|---|---|
| hsa-miR-575 | 479056_mir | GAGCCAGUUGGACAGGAGC (SEQ ID NO: 1) |

(continued)

| (Target microRNA) | Assay ID | Context Sequence |
|---|---|---|
| hsa-miR-3610 | 478053_mir | GAAUCGGAAAGGAGGCGCCG (SEQ ID NO: 2) |
| hsa-miR-3938 | 479757_mir | AAUUCCCUUGUAGAUAACCCGG (SEQ ID NO: 3) |
| hsa-miR-4476 | 478903_mir | CAGGAAGGAUUUAGGGACAGGC (SEQ ID NO: 4) |
| hsa-miR-4669 | 478925_mir | UGUGUCCGGGAAGUGGAGGAGG (SEQ ID NO: 5) |
| hsa-miR-5003-3p | 480076_mir | UACUUUUCUAGGUUGUUGGGG (SEQ ID NO: 6) |
| hsa-miR-5189-5p | 480104_mir | UCUGGGCACAGGCGGAUGGACAGG (SEQ ID NO: 7) |
| hsa-miR-6500-5p | 480192_mir | AGGAGCUAUCCACUCCAGGUGUCC (SEQ ID NO: 8) |
| hsa-miR-6511a-5p | 480208_mir | CAGGCAGAAGUGGGGCUGACAGG (SEQ ID NO: 9) |
| hsa-miR-6803-5p | 480373_mir | CUGGGGGUGGGGGGCUGGGCGU (SEQ ID NO: 10) |
| hsa-miR-6807-5p | 480380_mir | GUGAGCCAGUGGAAUGGAGAGG (SEQ ID NO: 11) |
| hsa-miR-6856-5p | 480468_mir | AAGAGAGGAGCAGUGGUGCUGUGG (SEQ ID NO: 12) |
| hsa-miR-6875-5p | 480501_mir | UGAGGGACCCAGGACAGGAGA (SEQ ID NO: 13) |

2. Result

(1) Analysis using miR-3610 and miR-4669 as normalization factors

As a result of performing the miRNA array analysis using the 8 cases of the comprehensive analysis cohort, a plurality of miRNAs that were significantly increased or decreased in the urine of the gastric cancer cases were extracted (Figs. 3 and 4).

Next, in the 190 cases of the training set, as a result of measuring each extracted miRNA by the qPCR method, miR-6807-5p and miR-6856-5p were identified as significant gastric cancer biomarkers, by multivariate analysis using miR-3610 and miR-4669 as normalization factors (Fig. 5). Using the gastric cancer biomarkers (miR-6807-5p and miR-6856-5p) and Helicobacter pylori infection status (positive), the diagnostic panel "a biomarker panel using the Helicobacter pylori infection status (positive) and expression of urinary biomarkers (miR-6807-5p and miR-6856-5p)" was constructed (Fig. 6).

Finally, when the accuracy of the diagnostic panel established in the training set was examined in 108 cases of the validation set (Fig. 7), AUC was 0.885 (95% confidence interval: 0.825-0.948), which was a very good result (Fig. 8).

As an example of a determination method using the Helicobacter pylori infection status (positive) and the expression of urinary biomarkers (miR-6807-5p and miR-6856-5p), the following simple model expression was created while considering the results of a multivariate analysis (logistic analysis). In both cases of being positive for Helicobacter pylori infection and being negative for Helicobacter pylori infection, when the value of the calculation expression of 0.68 or more was regarded as gastric cancer, the determination results of "sensitivity: 72.4% and specificity: 60.8%" were obtained. In the expression, $\Delta$Ct(miR-6807-5p) represents the $\Delta$Ct value of miR-6807-5p and $\Delta$Ct(miR-6856-5p) represents the $\Delta$Ct value of miR-6856-5p.

In a case of being positive for Helicobacter pylori infection: $65 \times 2^{-\Delta Ct(miR\text{-}6807\text{-}5p)} + 14 \times 2^{-\Delta Ct(miR\text{-}6856\text{-}5p)}$

In a case of being negative for Helicobacter pylori infection: $63 \times 2^{-\Delta Ct(miR\text{-}6807\text{-}5p)} + 5 \times 2^{-\Delta Ct(miR\text{-}6856\text{-}5p)}$

On the other hand, when a model expression ($-1.471 + 1.7486 \times (HP) + 63.424 \times 2^{-\Delta Ct(miR\text{-}6807\text{-}5p)} + 7.8641 \times 2^{-\Delta Ct(miR\text{-}6856\text{-}5p)}$) (here, in the expression, HP is 1 in a case of being positive for Helicobacter pylori infection and is 0 in a case of being negative for Helicobacter pylori infection) is created from the results of the multivariate analysis (logistic analysis) and it is assumed that the prevalence of gastric cancer in Japanese 60 years old and over 60 years old is 0.2% from the past statistics, with the model expression, the risk can be classified into three groups: A (low risk of gastric cancer), B (average risk of gastric cancer), and C (high risk of gastric cancer) (Fig. 9). It is considered that the C group can be used for determination such as a need for secondary scrutiny.

(2) Analysis using miR-3610, miR-4669, and miR-6803-5p as normalization factors

When using three miRNAs including, in addition to miR-3610 and miR-4669, miR-6803-5p as a next candidate for normalization, the significant biomarkers with the Helicobacter pylori infection status (positive) in the analysis using the training set and miR-575 were extracted as significant biomarkers (Figs. 10 and 11).

When the accuracy of "biomarker panel using the Helicobacter pylori infection status (positive) and expression of urinary biomarker miR-575" was examined in 108 cases of the validation set, AUC was 0.830 (95% confidence interval: 0.752-0.907), which was a very good result (Figs. 12 and 13).

As an example of the determination method, as (above) in the case of using miR-6807-5p and miR-6856-5p as the biomarkers, the following conditions were created while considering the results of the multivariate analysis (logistic analysis). As a result, it was possible to determine as "sensitivity: 67.1% and specificity: 51.7%". In the expression, ΔCt(miR-575) represents the ΔCt value of miR-575.

<Conditions> In a case where the following mathematical expression is satisfied, it is determined that "a person has gastric cancer".

In a case of being positive for Helicobacter pylori infection : $2^{-\Delta Ct(miR\text{-}575)} > 0.03446916$

In a case of being negative for Helicobacter pylori infection: $2^{-\Delta Ct(miR\text{-}575)} > 0.02673549$

On the other hand, when a model expression ($-1.2062 + 2.2727 \times (HP) + 18.4419 \times 2^{-\Delta Ct(miR\text{-}575)}$) (here, in the expression, HP is 1 in a case of being positive for Helicobacter pylori infection and is 0 in a case of being negative for Helicobacter pylori infection) is created from the results of the multivariate analysis (logistic analysis) and it is assumed that the prevalence of gastric cancer in Japanese 60 years old and over 60 years old is 0.2% from the past statistics, with the model expression, the risk can be classified into three groups: A (low risk of gastric cancer), B (average risk of gastric cancer), and C (high risk of gastric cancer) (Fig. 14). It is considered that the C group can be used for determination such as a need for secondary scrutiny.

(3) Detection of biomarkers in other body fluids

It was estimated that three types of miRNAs of miR-6807-5p, miR-6856-5p, and miR-575, which were significantly increased in the urine of gastric cancer patients, can be used as biomarkers even when other body fluids such as serum were used as samples. Therefore, the following verification experiment was conducted.

The expressions of miR-6807-5p, miR-6856-5p, and miR-575 were measured by qPCR, using the serum of 64 cases (gastric cancer and healthy people: 32 cases respectively) matched by age and gender. The measurement method was based on the above experiment.

Even in both cases of normalization using two types of miRNA (miR-3610 and miR-4669) and normalization using three types of miRNA (miR-3610, miR-4669, and miR-6803-5p), it was confirmed that miR-6807-5p, miR-6856-5p, and miR-575 were significantly highly expressed in the serum of gastric cancer patients as compared with healthy people (Figs. 15 to 18). This result shows that three types of miRNAs (miR-6807-5p, miR-6856-5p, and miR-575) are useful as gastric cancer-specific biomarkers not only in urine but also in serum or other body fluid samples. miR-3610, miR-4669, and miR-6803-5p used as the normalization factors are constitutively and stably expressed in urine, the serum, and the like, and are thus useful as normalization factors for miRNAs in body fluids.

(4) Detection/diagnosis of early gastric cancer and determination of efficacy of gastric cancer treatment

In order to verify whether the biomarkers (miR-6807-5p and miR-6856-5p) are effective in detecting and diagnosing early gastric cancer, comparative examination between healthy people (149 cases) in the training set and validation set and early gastric cancer cases of stage I (95 cases) was conducted by using the urine samples. As a result, it was confirmed that both miR-6807-5p and miR-6856-5p were highly expressed in the urine of early gastric cancer patients at stage I, as compared with the healthy people (Fig. 19). Also, in ROC analysis, "biomarker panel using positive Helicobacter pylori infection and expression of miR-6807-5p/miR-6856-5p in urine" satisfies AUC = 0.748 (95% confidence interval: 0.683-0.812), and healthy people and early gastric cancer patients were well discriminated (Fig. 20). Furthermore, the expression levels of the two types of the urinary biomarkers (miR-6807-5p and miR-6856-5p) both showed a significant positive correlation with clinical stage (Fig. 21). In the expression level analysis of two types of biomarkers (miR-6807-5p and miR-6856-5p) in tissues, miR-6807-5p showed significantly higher expression in gastric cancer tissues than in normal tissues, and although the number of cases was small and not significant, miR-6856-5p also showed a tendency for high expression in gastric cancer tissues compared to normal tissues (Fig. 22). Furthermore, when urinary miRNA expression analysis was performed before and after resection of gastric cancer, it was confirmed that all urinary biomarkers (miR-6807-5p and miR-6856-5p) showed a significant and remarkable decrease in expression after resection of gastric cancer (Fig. 23), and it was shown that all urinary biomarkers reflect the efficacy of a gastric cancer treatment very well.

[Industrial Applicability]

The present invention provides a biomarker useful for the diagnosis of gastric cancer. The biomarker of the present invention can be detected in a sample such as urine or serum, and enables a minimally invasive and simple test. In particular, in a case of using the urine as a sample, test and diagnosis can be performed "non-invasively", "easily", and "at home", which is extremely useful in the field of primary screening, for example. Moreover, if the biomarker of the present invention is used, highly accurate test becomes possible.

The present invention can be carried out, for example, as an item of urinalysis in a health checkup performed in a hospital, a health center, a medical examination vehicle, or the like. In addition, when urine is used as a sample, a new style of test is possible, in which urine collected at home is sent to a testing institution to be tested.

The present invention is not limited to the description of the embodiments and examples of the invention. Various modifications are also included in the present invention as long as those skilled in the art can easily conceive of without departing from the description of claims. The entire contents of the papers, unexamined patent application publications, and patent publications, specified in the present specification are cited by reference.

## Claims

1. A gastric cancer biomarker, comprising:
one microRNA or a combination of two or more microRNAs selected from the group consisting of hsa-miR-6807-5p, hsa-miR-6856-5p, and hsa-miR-575.

2. The gastric cancer biomarker according to Claim 1,
wherein the gastric cancer biomarker is detected in a urine sample or a blood sample.

3. A test method for gastric cancer, in which an expression level of the gastric cancer biomarker according to Claim 1 or 2 is used as an index.

4. The gastric cancer biomarker according to Claim 1 or 2, comprising:

   hsa-miR-6807-5p or hsa-miR-6856-5p; or
   a combination of hsa-miR-6807-5p and hsa-miR-6856-5p,
   wherein the gastric cancer biomarker is used for detecting early gastric cancer.

5. A test method for early gastric cancer, in which an expression level of the gastric cancer biomarker according to Claim 4 is used as an index.

6. The gastric cancer biomarker according to Claim 1 or 2, comprising:

   hsa-miR-6807-5p or hsa-miR-6856-5p; or
   a combination of hsa-miR-6807-5p and hsa-miR-6856-5p,
   wherein the gastric cancer biomarker is used for determining the efficacy of a gastric cancer treatment or predicting gastric cancer recurrence.

7. The method according to Claim 3, comprising the steps of (1) to (3) below:

   (1) a step of detecting the gastric cancer biomarker in a sample collected from a subject and determining an expression level;
   (2) a step of obtaining a result of a Helicobacter pylori infection test of the subject; and
   (3) a step of determining morbidity of gastric cancer, based on the expression level determined in the step (1) and the result of the Helicobacter pylori infection test prepared in the step (2).

8. The method according to Claim 7,
wherein the gastric cancer biomarker to be detected in the step (1) is hsa-miR-6807-5p and hsa-miR-6856-5p, and the expression level of the gastric cancer biomarker is determined by normalization using hsa-miR-3610 and hsa-miR-4669 as normalization factors.

9. The method according to Claim 7,
wherein the gastric cancer biomarker to be detected in the step (1) is hsa-miR-575, and
the expression level of the gastric cancer biomarker is determined by normalization using hsa-miR-3610, hsa-miR-4669, and hsa-miR-6803-5p as normalization factors.

10. The method according to any one of Claims 7 to 9,
wherein the level of the gastric cancer biomarker has a positive correlation with the morbidity of gastric cancer.

11. The method according to any one of Claims 7 to 10,
wherein the gastric cancer biomarker is detected by a qRT-PCR method.

**12.** The method according to Claim 11,
wherein, after obtaining a value (ΔCt value) obtained by subtracting a Ct value of a normalization factor from a Ct value of the gastric cancer biomarker, the morbidity of gastric cancer is determined using two mathematical expressions with the ΔCt value as a variable, the two mathematical expressions being set separately for a case where the subject is positive for Helicobacter pylori infection and a case where the subject is negative for Helicobacter pylori infection.

**13.** The method according to Claim 5, comprising the steps of (1) to (3) below:

(1) a step of detecting the gastric cancer biomarker in a sample collected from a subject and determining an expression level;
(2) a step of obtaining a result of a Helicobacter pylori infection test of the subject; and
(3) a step of determining morbidity of early gastric cancer, based on the expression level determined in the step (1) and the result of the Helicobacter pylori infection test prepared in the step (2).

**14.** The method according to Claim 13,
wherein the gastric cancer biomarker to be detected in the step (1) is hsa-miR-6807-5p and hsa-miR-6856-5p, and the expression level of the gastric cancer biomarker is determined by normalization using hsa-miR-3610 and hsa-miR-4669 as normalization factors.

**15.** The method according to Claim 13 or 14,
wherein the level of the gastric cancer biomarker has a positive correlation with a stage of gastric cancer.

**16.** The method according to any one of Claims 13 to 15,
wherein the gastric cancer biomarker is detected by a qRT-PCR method.

**17.** The method according to Claim 16,
wherein, after obtaining a value (ΔCt value) obtained by subtracting a Ct value of a normalization factor from a Ct value of the gastric cancer biomarker, the morbidity of early gastric cancer is determined using two mathematical expressions with the ΔCt value as a variable, the two mathematical expressions being set separately for a case where the subject is positive for Helicobacter pylori infection and a case where the subject is negative for Helicobacter pylori infection.

**18.** The method according to any one of Claims 7 to 17,
wherein the sample is urine or blood.

**19.** A gastric cancer test kit, comprising:

a reagent for detecting the gastric cancer biomarker according to Claim 1; and
an instruction manual.

**20.** A gastric cancer test kit, comprising:

a reagent for detecting the gastric cancer biomarker according to Claim 4 or 6; and
an instruction manual.

**21.** The gastric cancer test kit according to Claim 19 or 20,
wherein the reagent is a combination of a reagent for detecting hsa-miR-6807-5p and a reagent for detecting hsa-miR-6856-5p.

**22.** The gastric cancer test kit according to Claim 19,
wherein the reagent is a reagent for detecting hsa-miR-575.

**23.** The gastric cancer test kit according to any one of Claims 19 to 22, further comprising:
one or more elements selected from the group consisting of a reagent for extracting a microRNA, a normalization factor-specific primer, a DNA polymerase, a reverse transcriptase, dNTPs, a urine sample collection container, a reactor, and a detector.

FIG. 1

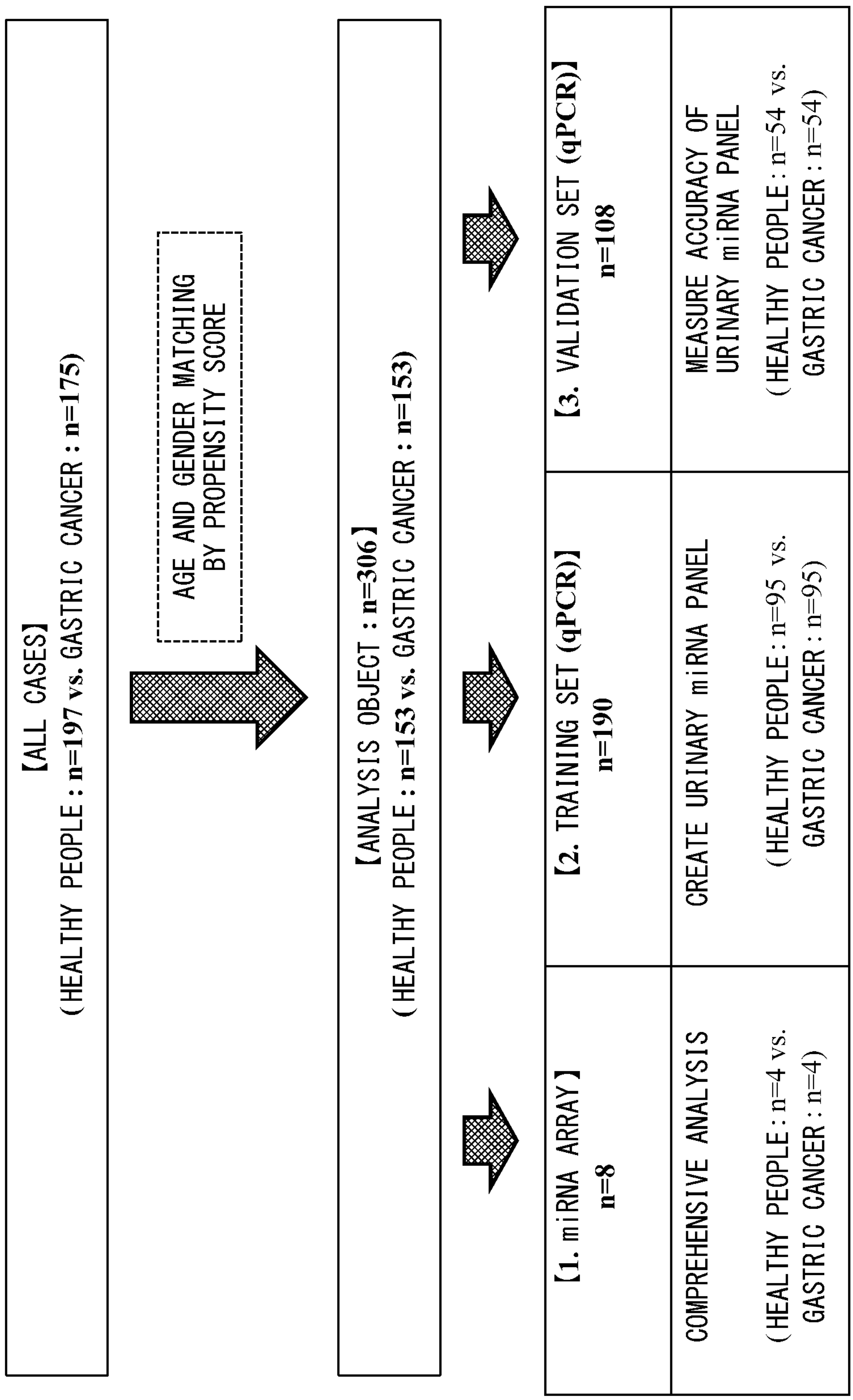
【ALL CASES】
(HEALTHY PEOPLE : n=197 vs. GASTRIC CANCER : n=175)
AGE AND GENDER MATCHING BY PROPENSITY SCORE
【ANALYSIS OBJECT : n=306】
(HEALTHY PEOPLE : n=153 vs. GASTRIC CANCER : n=153)
【1. miRNA ARRAY】
n=8
COMPREHENSIVE ANALYSIS
(HEALTHY PEOPLE : n=4 vs. GASTRIC CANCER : n=4)
【2. TRAINING SET (qPCR)】
n=190
CREATE URINARY miRNA PANEL
(HEALTHY PEOPLE : n=95 vs. GASTRIC CANCER : n=95)
【3. VALIDATION SET (qPCR)】
n=108
MEASURE ACCURACY OF URINARY miRNA PANEL
(HEALTHY PEOPLE : n=54 vs. GASTRIC CANCER : n=54)

FIG. 2

| | | HEALTHY PEOPLE (n=153) | GASTRIC CANCER (n=153) | p VALUE |
|---|---|---|---|---|
| GENDER | MALE | 109 | 110 | 0.899 |
| | FEMALE | 44 | 43 | |
| AGE (MEDIAN) | | 68 (38-88) | 70 (42-85) | 0.077 |
| SERUM CREATININE(mg/dl, AVERAGE±SD) | | 0.79±0.16 | 0.80±0.24 | 0.753 |
| HP status | POSITIVE | 21 | 74 | |
| | NEGATIVE (INCLUDING AFTER STERILIZATION) | 132 | 76 | <0.001 |
| | UNCLEAR | 0 | 3 | |
| Stage I | | | 97 | |
| Stage II | | | 16 | |
| Stage III | | | 15 | |
| Stage IV | | | 25 | |

# FIG. 3

| miRNA NAME | | MAGNIFICATION CHANGE (GASTRIC CANCER VS. HEALTHY PEOPLE) | |
|---|---|---|---|
| hsa-miR-1185-2-3p | | 6.923106 | up |
| hsa-miR-4674 | | 6.354258 | up |
| hsa-miR-4447 | | 6.056814 | up |
| hsa-miR-1233-5p | | 5.512341 | up |
| hsa-miR-3181 | | 5.190352 | up |
| hsa-miR-4535 | | 4.927193 | up |
| hsa-miR-6856-5p | * | 4.523265 | up |
| hsa-miR-4638-3p | | 4.506600 | up |
| hsa-miR-6511a-5p | * | 4.482375 | up |
| hsa-miR-575 | * | 4.402103 | up |
| hsa-miR-3938 | * | 4.160178 | up |
| hsa-miR-6875-5p | * | 4.145202 | down |
| hsa-miR-6845-5p | | 3.810419 | down |
| hsa-miR-4746-3p | | 3.792608 | up |
| hsa-miR-3605-5p | | 3.614511 | down |
| hsa-miR-3620-5p | | 3.530000 | up |
| hsa-miR-6807-5p | * | 3.393903 | up |
| hsa-miR-4476 | * | 3.385409 | up |
| hsa-miR-5003-3p | * | 3.275088 | up |
| hsa-miR-1469 | | 3.213763 | up |
| hsa-miR-6500-5p | * | 3.085568 | down |
| hsa-miR-5189-5p | * | 3.042273 | down |
| hsa-miR-601 | | 2.960206 | up |

FIG. 4

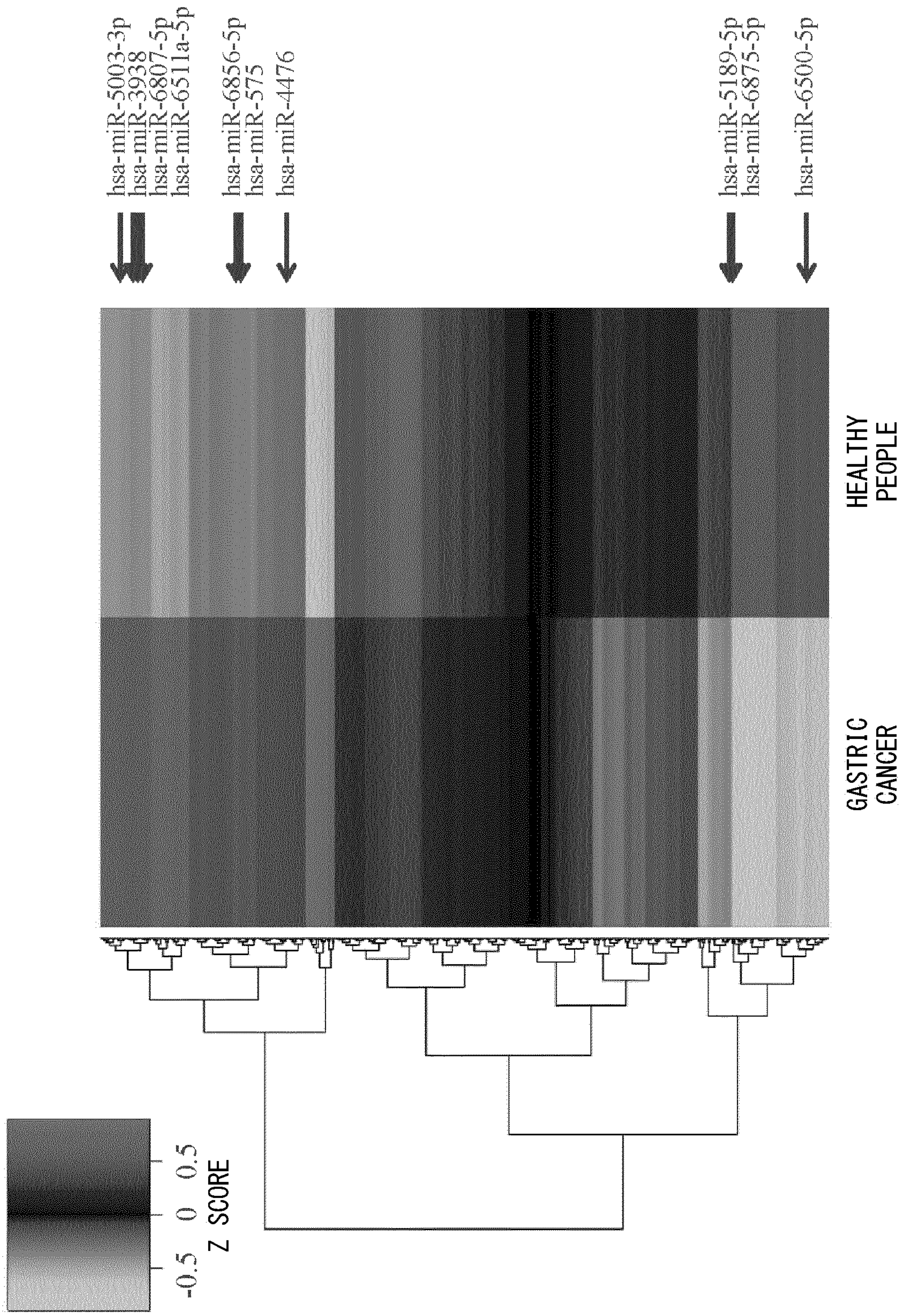

hsa-miR-5003-3p
hsa-miR-3938
hsa-miR-6807-5p
hsa-miR-6511a-5p
hsa-miR-6856-5p
hsa-miR-575
hsa-miR-4476
hsa-miR-5189-5p
hsa-miR-6875-5p
hsa-miR-6500-5p
HEALTHY PEOPLE
GASTRIC CANCER
-0.5 0 0.5
Z SCORE

# FIG. 5

| | $2^{-\Delta Ct}$ (AVERAGE VALUE ± SD)¶ | | UNIVARIATE ANALYSIS | MULTIVARIATE ANALYSIS | | |
|---|---|---|---|---|---|---|
| | HEALTHY PEOPLE (n=95) | GASTRIC CANCER (n=95) | p VALUE | ODDS RATIO (95% CONFIDENCE INTERVAL) | | p VALUE |
| **miR-575** | 0.013±0.008 | 0.025±0.030 | <0.001 | | | |
| **miR-3938** | 0.314±0.153 | 0.428±0.362 | 0.005 | | | |
| **miR-4476** | 0.032±0.024 | 0.035±0.029 | 0.339 | | | |
| **miR-5003-3p** | 0.237±0.232 | 0.358±0.441 | 0.019 | | | |
| **miR-5189-5p** | 0.219±0.897 | 0.198±0.629 | 0.853 | | | |
| **miR-6500-5p** | 0.548±2.024 | 0.476±1.220 | 0.770 | | | |
| **miR-6511a-5p** | 0.013±0.012 | 0.022±0.034 | 0.018 | | | |
| **miR-6807-5p** | 0.011±0.008 | 0.017±0.016 | <0.001 | 1.580 | (1.06-2.34) | 0.024 |
| **miR-6856-5p** | 0.024±0.015 | 0.040±0.042 | <0.001 | 1.210 | (1.01-1.46) | 0.042 |
| **miR-6875-5p** | 0.069±0.036 | 0.099±0.103 | 0.008 | | | |
| HP POSITIVE (n) | 12 | 48 | <0.001 | 5.82 | (2.71-12.5) | <0.001 |

FIG. 6

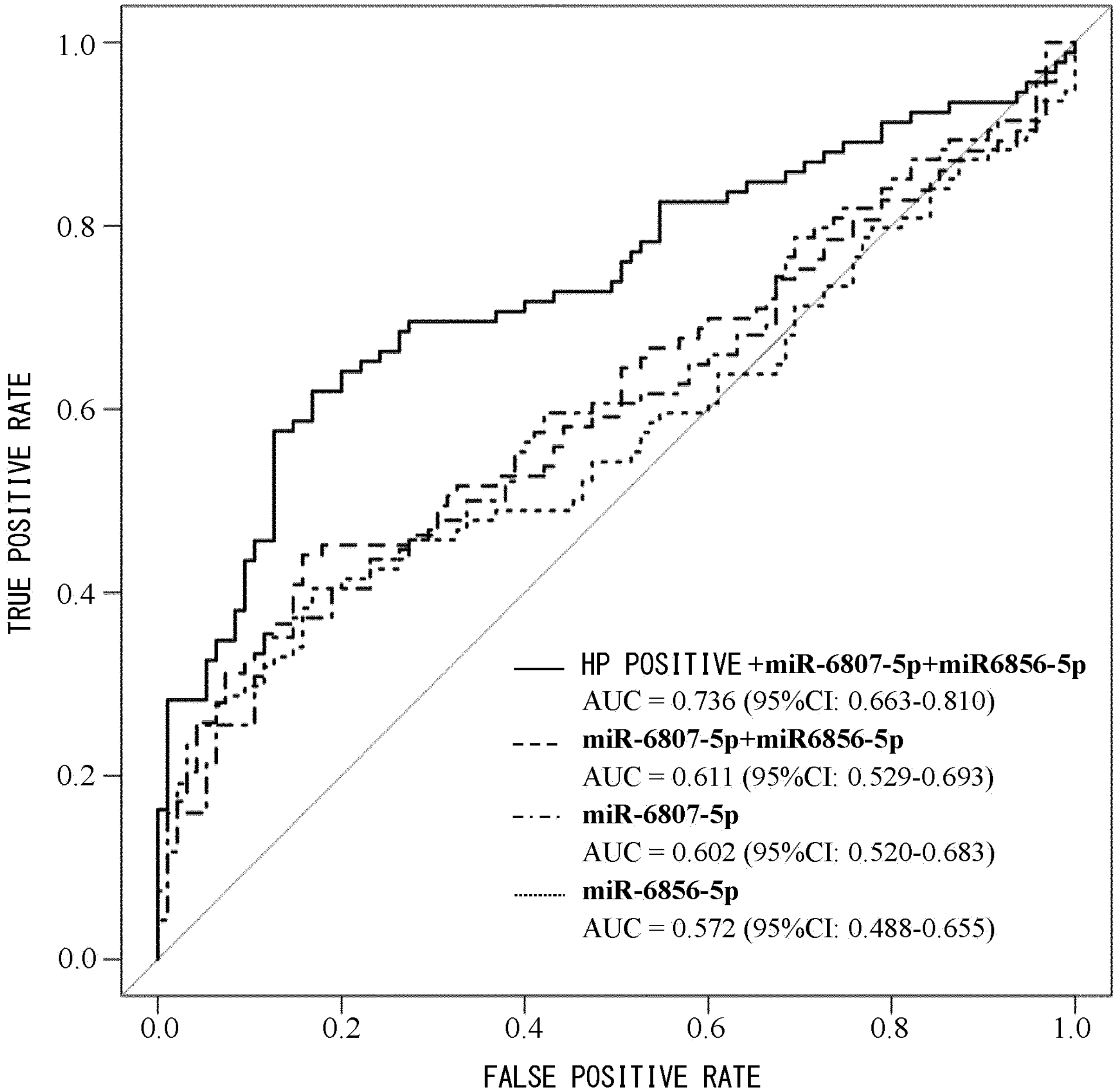
TRUE POSITIVE RATE
FALSE POSITIVE RATE
0.0
0.2
0.4
0.6
0.8
1.0
HP POSITIVE+miR-6807-5p+miR6856-5p
AUC = 0.736 (95%CI: 0.663-0.810)
miR-6807-5p+miR6856-5p
AUC = 0.611 (95%CI: 0.529-0.693)
miR-6807-5p
AUC = 0.602 (95%CI: 0.520-0.683)
miR-6856-5p
AUC = 0.572 (95%CI: 0.488-0.655)

FIG. 7

| | $2^{-\Delta Ct}$ (AVERAGE VALUE ± SD)¶ | | UNIVARIATE ANALYSIS | MULTIVARIATE ANALYSIS | |
|---|---|---|---|---|---|
| | HEALTHY PEOPLE (n=54) | GASTRIC CANCER (n=54) | p VALUE | ODDS RATIO (95% CONFIDENCE INTERVAL) | p VALUE |
| **miR-6807-5p** | 0.004±0.005 | 0.015±0.010 | <0.001 | 19.40 (4.61-81.90) | <0.001 |
| **miR-6856-5p** | 0.018±0.022 | 0.035±0.040 | 0.005 | 0.793 (0.565-1.11) | 0.178 |
| HP POSITIVE (n) | 12 | 48 | <0.001 | 3.26 (1.03-10.30) | 0.044 |

FIG. 8

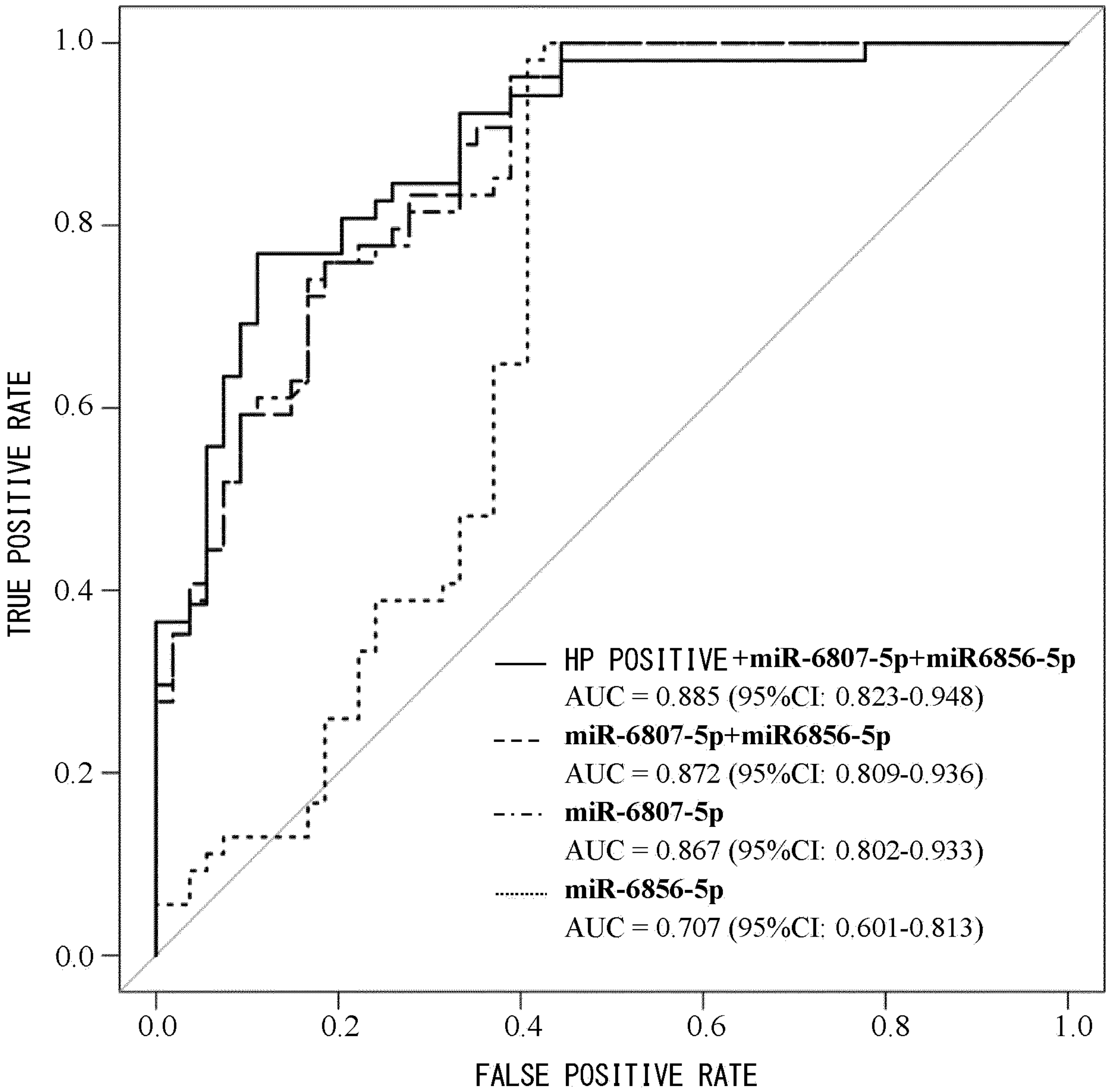
1.0
0.8
0.6
0.4
0.2
0.0
TRUE POSITIVE RATE
0.0
0.2
0.4
0.6
0.8
1.0
FALSE POSITIVE RATE
HP POSITIVE+miR-6807-5p+miR6856-5p
AUC = 0.885 (95%CI: 0.823-0.948)
miR-6807-5p+miR6856-5p
AUC = 0.872 (95%CI: 0.809-0.936)
miR-6807-5p
AUC = 0.867 (95%CI: 0.802-0.933)
miR-6856-5p
AUC = 0.707 (95%CI: 0.601-0.813)

FIG. 9

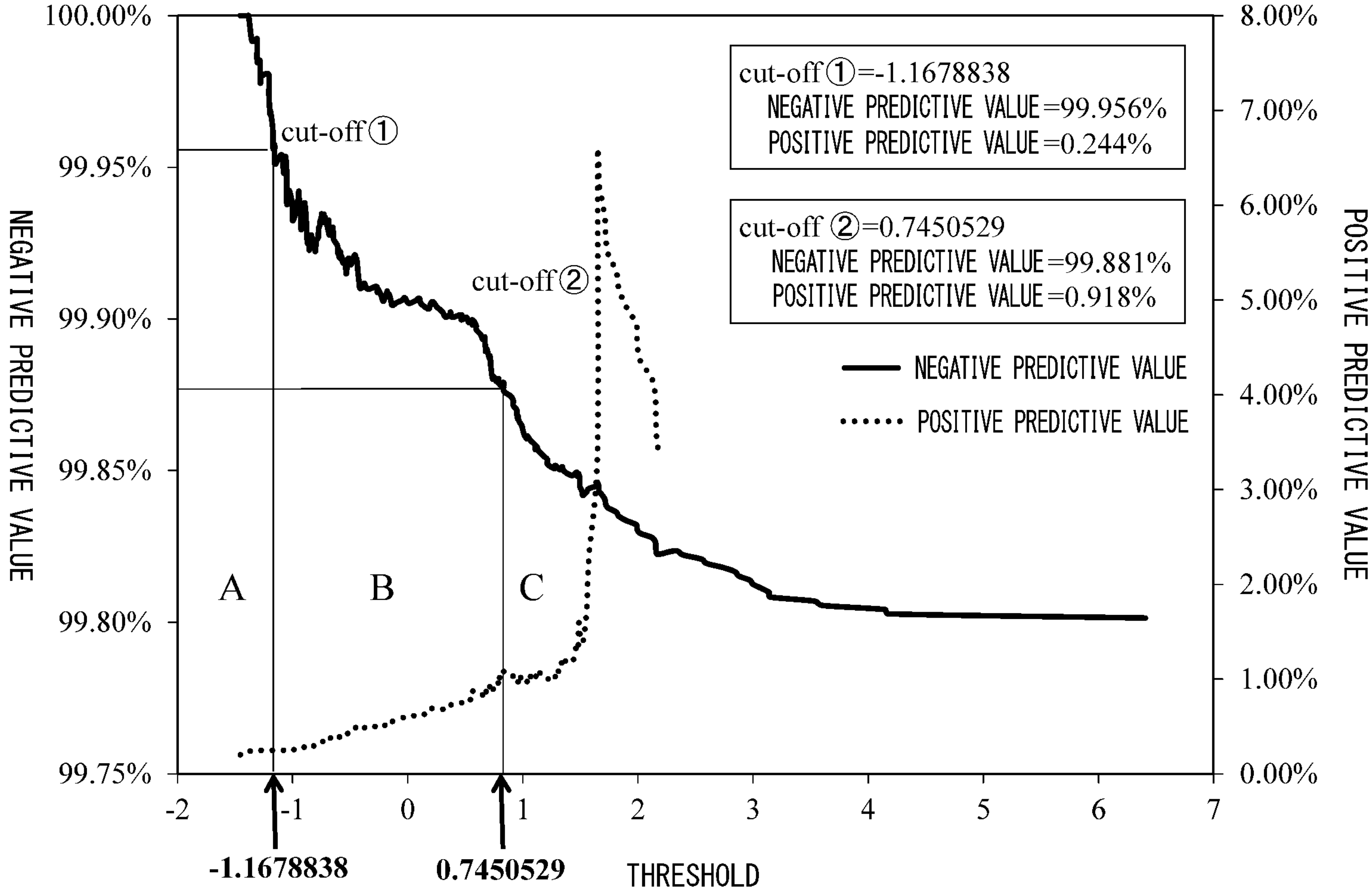
cut-off①=-1.1678838
NEGATIVE PREDICTIVE VALUE=99.956%
POSITIVE PREDICTIVE VALUE=0.244%
cut-off ②=0.7450529
NEGATIVE PREDICTIVE VALUE=99.881%
POSITIVE PREDICTIVE VALUE=0.918%
NEGATIVE PREDICTIVE VALUE
POSITIVE PREDICTIVE VALUE
cut-off①
cut-off②
A
B
C
100.00%
99.95%
99.90%
99.85%
99.80%
99.75%
NEGATIVE PREDICTIVE VALUE
8.00%
7.00%
6.00%
5.00%
4.00%
3.00%
2.00%
1.00%
0.00%
POSITIVE PREDICTIVE VALUE
-2
-1
0
1
2
3
4
5
6
7
-1.1678838
0.7450529
THRESHOLD

# FIG. 10

| | $2^{-\Delta Ct}$ (AVERAGE VALUE ± SD)¶ | | UNIVARIATE ANALYSIS | MULTIVARIATE ANALYSIS | |
|---|---|---|---|---|---|
| | HEALTHY PEOPLE (n=95) | GASTRIC CANCER (n=95) | p VALUE | ODDS RATIO (95% CONFIDENCE INTERVAL) | p VALUE |
| **miR-575** | 0.034±0.023 | 0.052±0.049 | <0.001 | 1.160 (1.020-1.320) | 0.024 |
| **miR-3938** | 6.377±3.443 | 0.966±0.694 | 0.096 | | |
| **miR-4476** | 0.526±0.359 | 0.076±0.057 | 0.841 | | |
| **miR-5003-3p** | 0.558±0.465 | 0.692±0.699 | 0.121 | | |
| **miR-5189-5p** | 0.407±1.697 | 0.297±0.981 | 0.586 | | |
| **miR-6500-5p** | 0.995±3.313 | 0.782±2.044 | 0.600 | | |
| **miR-6511a-5p** | 0.034±0.023 | 0.046±0.054 | 0.055 | | |
| **miR-6807-5p** | 0.026±0.016 | 0.034±0.026 | 0.012 | | |
| **miR-6856-5p** | 0.064±0.032 | 0.082±0.064 | 0.011 | | |
| **miR-6875-5p** | 0.181±0.087 | 0.208±0.166 | 0.171 | | |
| HP POSITIVE (n) | 12 | 48 | <0.001 | 5.650 (2.66-12.00) | <0.001 |

FIG. 11

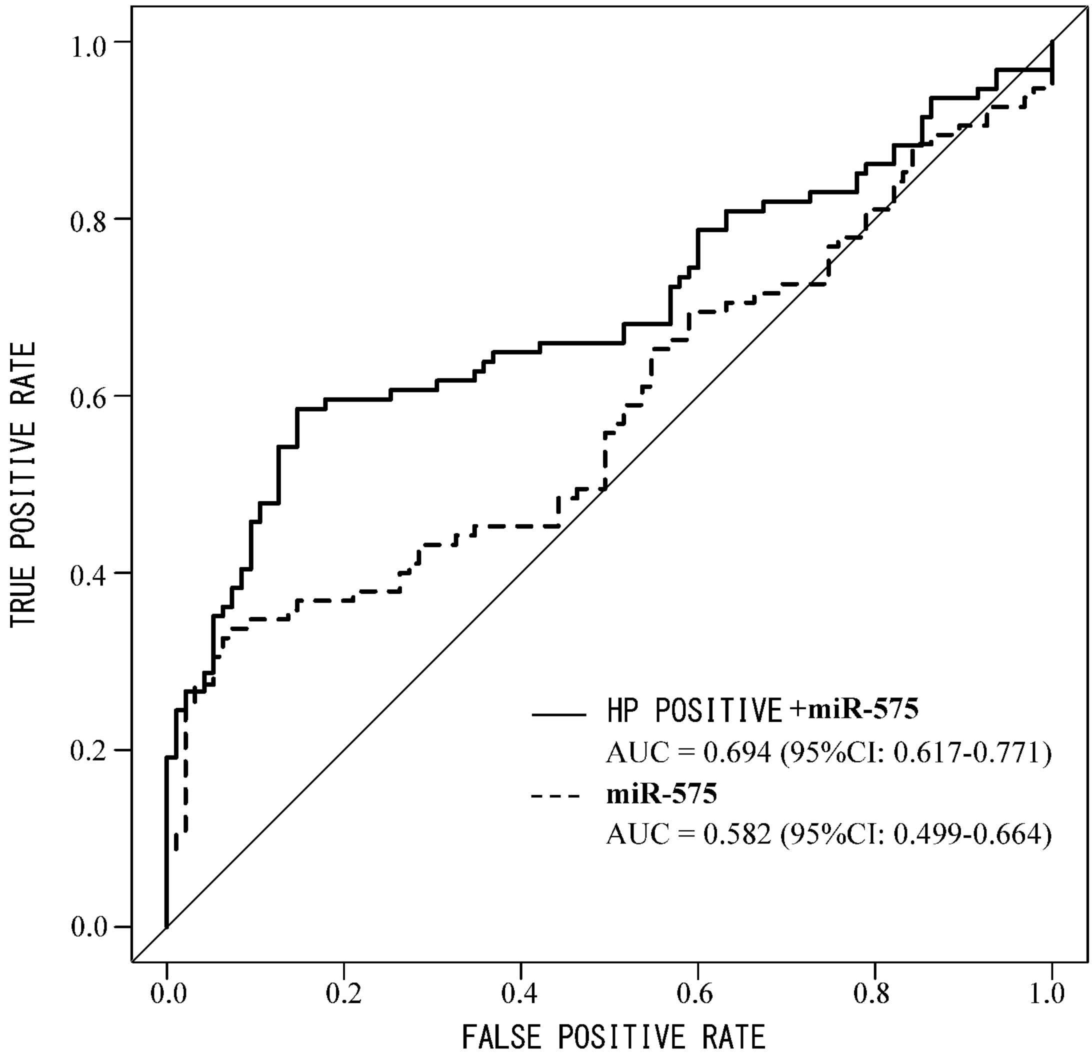
TRUE POSITIVE RATE
1.0
0.8
0.6
0.4
0.2
0.0
0.0
0.2
0.4
0.6
0.8
1.0
FALSE POSITIVE RATE
HP POSITIVE +miR-575
AUC = 0.694 (95%CI: 0.617-0.771)
miR-575
AUC = 0.582 (95%CI: 0.499-0.664)

FIG. 12

| | $2^{-\Delta Ct}$ (AVERAGE VALUE ± SD)¶ | | UNIVARIATE ANALYSIS | MULTIVARIATE ANALYSIS | | |
|---|---|---|---|---|---|---|
| | HEALTHY PEOPLE (n=54) | GASTRIC CANCER (n=54) | p VALUE | ODDS RATIO (95% CONFIDENCE INTERVAL) | | p VALUE |
| **miR-575** | 0.008±0.010 | 0.020±0.013 | <0.001 | 19.40 | (4.61-81.90) | <0.001 |
| HP POSITIVE (n) | 12 | 48 | <0.001 | 3.26 | (1.03-10.30) | 0.044 |

FIG. 13

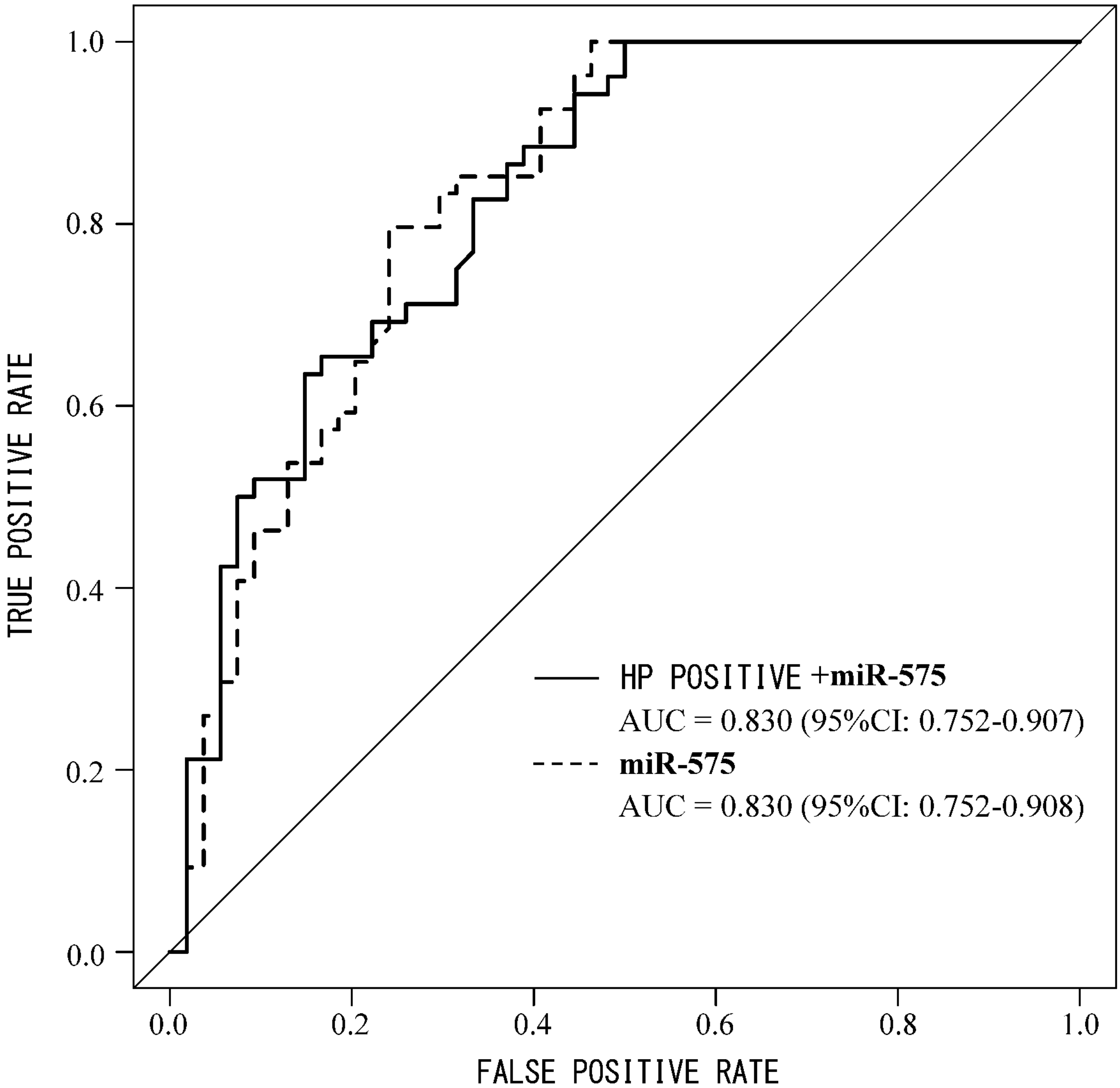
1.0
0.8
0.6
0.4
0.2
0.0
TRUE POSITIVE RATE
0.0
0.2
0.4
0.6
0.8
1.0
FALSE POSITIVE RATE
HP POSITIVE +miR-575
AUC = 0.830 (95%CI: 0.752-0.907)
miR-575
AUC = 0.830 (95%CI: 0.752-0.908)

FIG. 14

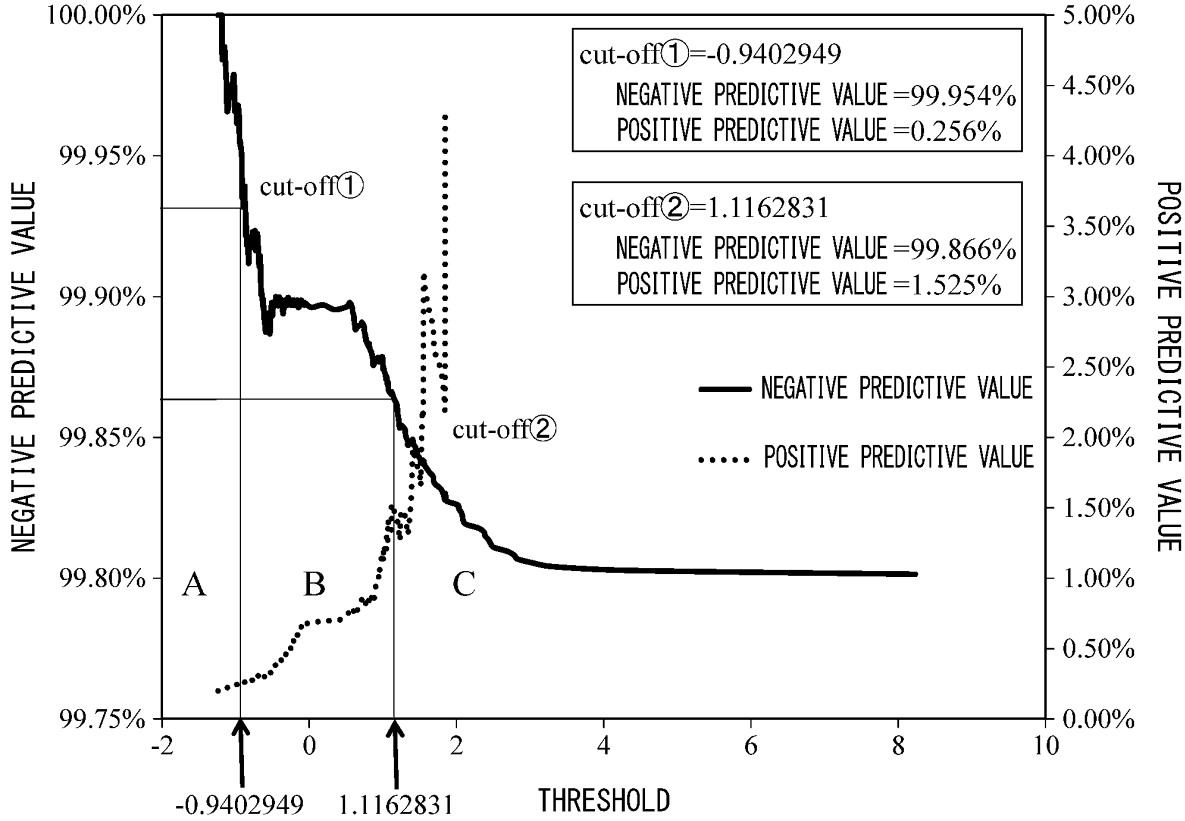


FIG. 15

NORMALIZATION USING miR-3610 + miR-4669

| | $2^{-\Delta Ct}$ (MEDIAN(INTERQUARTILE RANGE)) | | |
|---|---|---|---|
| | HEALTHY **(n=32)** | GASTRIC CANCER **(n=32)** | p VALUE |
| **miR-575** | 0.0008 (0.0004-0.0012) | 0.0017 (0.0010-0.0027) | **0.001** |
| **miR-6807-5p** | 0.0005 (0.0003-0.0008) | 0.0010 (0.0006-0.0012) | **0.003** |
| **miR-6856-5p** | 0.0009 (0.0007-0.0013) | 0.0015 (0.0011-0.0023) | **0.002** |
| HP POSITIVE (n) | | | **<0.001** |

FIG. 16

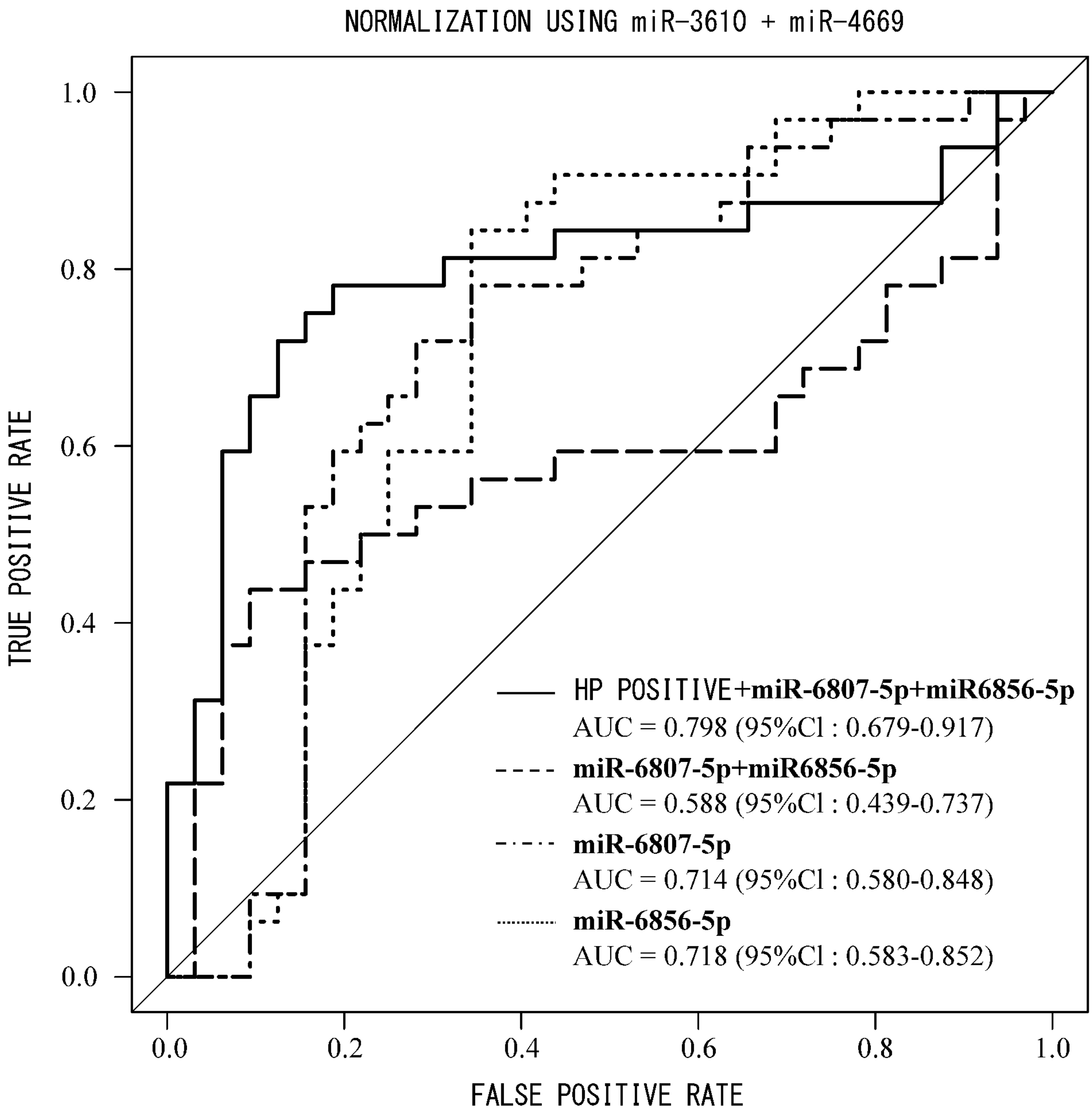
NORMALIZATION USING miR-3610 + miR-4669
1.0
0.8
0.6
0.4
0.2
0.0
TRUE POSITIVE RATE
0.0
0.2
0.4
0.6
0.8
1.0
FALSE POSITIVE RATE
HP POSITIVE+miR-6807-5p+miR6856-5p
AUC = 0.798 (95%Cl : 0.679-0.917)
miR-6807-5p+miR6856-5p
AUC = 0.588 (95%Cl : 0.439-0.737)
miR-6807-5p
AUC = 0.714 (95%Cl : 0.580-0.848)
miR-6856-5p
AUC = 0.718 (95%Cl : 0.583-0.852)

FIG. 17

NORMALIZATION USING miR-3610 + miR-4669 + miR-6803-5p

| | $2^{-\Delta Ct}$ (MEDIAN(INTERQUARTILE RANGE)) | | |
|---|---|---|---|
| | HEALTHY **(n=32)** | GASTRIC CANCER **(n=32)** | p VALUE |
| **miR-575** | 0.0080 (0.0052-0.0099) | 0.0120 (0.0073-0.0175) | **0.009** |
| **miR-6807-5p** | 0.0046 (0.0036-0.0061) | 0.0069 (0.0046-0.0083) | **0.021** |
| **miR-6856-5p** | 0.0083 (0.0065-0.0117) | 0.0098 (0.0086-0.0135) | **0.035** |
| HP POSITIVE (n) | | | **<0.001** |

FIG. 18

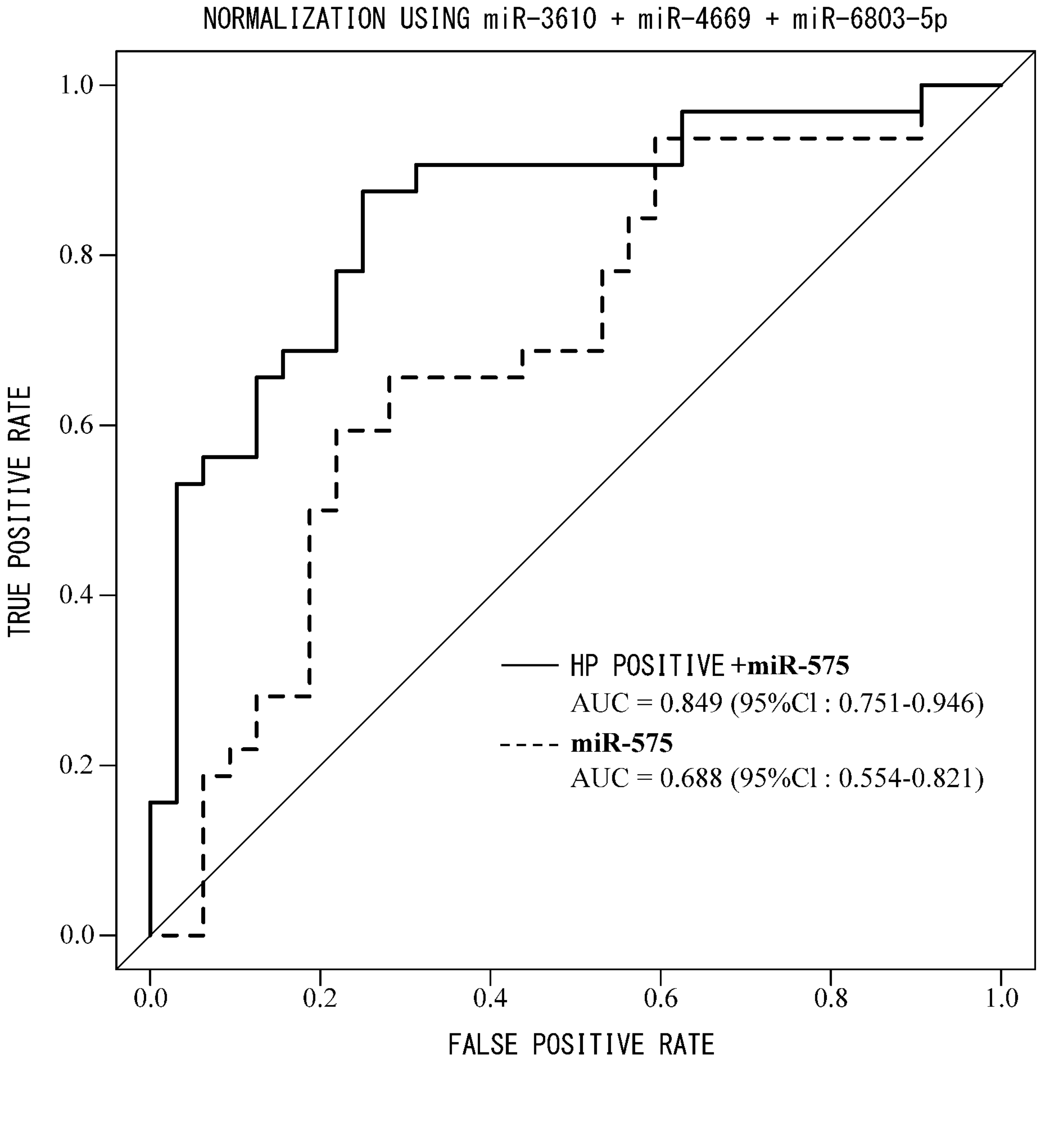
NORMALIZATION USING miR-3610 + miR-4669 + miR-6803-5p
1.0
0.8
0.6
0.4
0.2
0.0
TRUE POSITIVE RATE
0.0
0.2
0.4
0.6
0.8
1.0
FALSE POSITIVE RATE
HP POSITIVE +miR-575
AUC = 0.849 (95%Cl : 0.751-0.946)
miR-575
AUC = 0.688 (95%Cl : 0.554-0.821)

FIG. 19

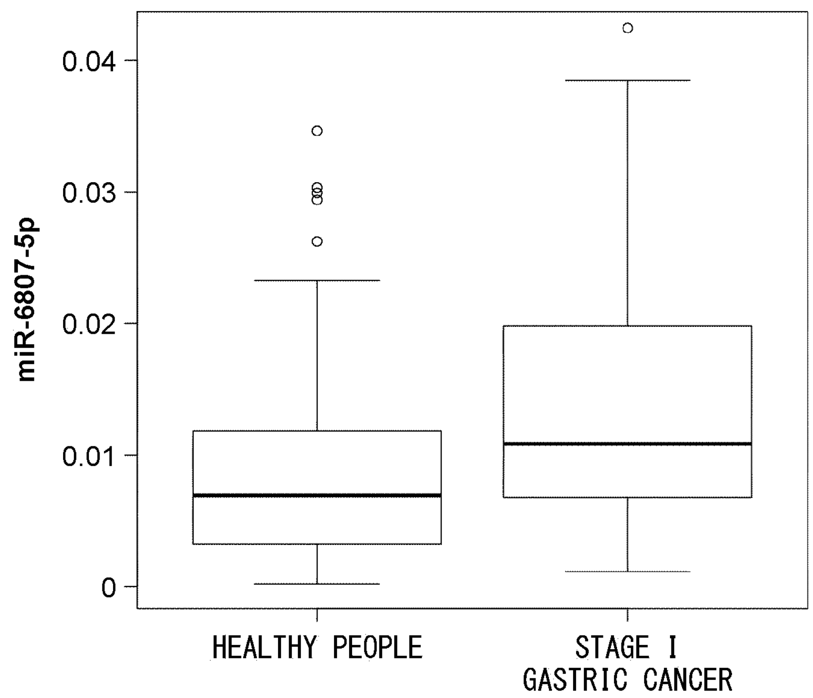


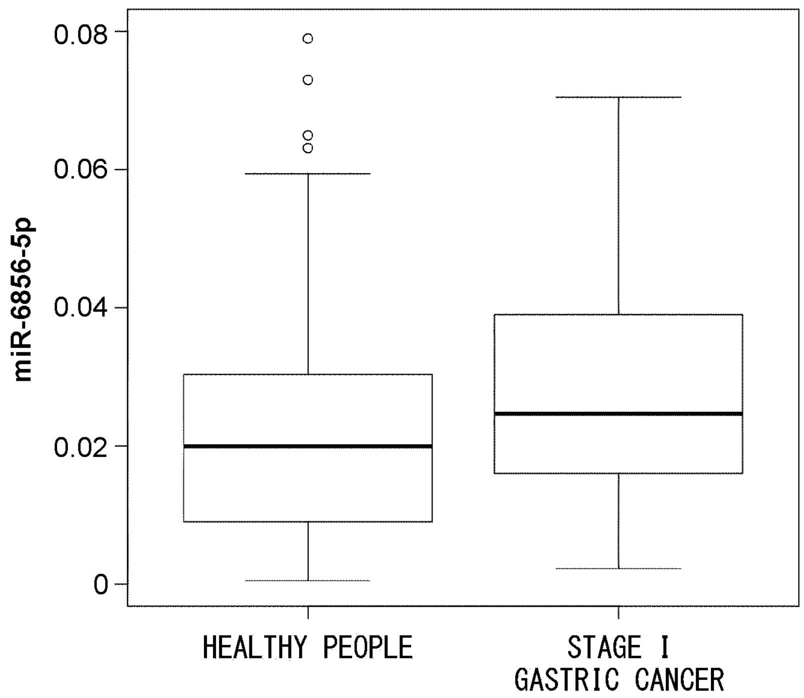


| | | HEALTHY PEOPLE **N=149** | STAGE I GASTRIC CANCER **N=95** | p VALUE |
|---|---|---|---|---|
| miR-6807-5p | MEDIAN (IQR) | 0.007 (0.003-0.012) | 0.011 (0.007-0.020) | **< 0.001** |
| miR-6856-5p | MEDIAN (IQR) | 0.020 (0.009-0.030) | 0.025 (0.016-0.039) | **< 0.001** |

FIG. 20

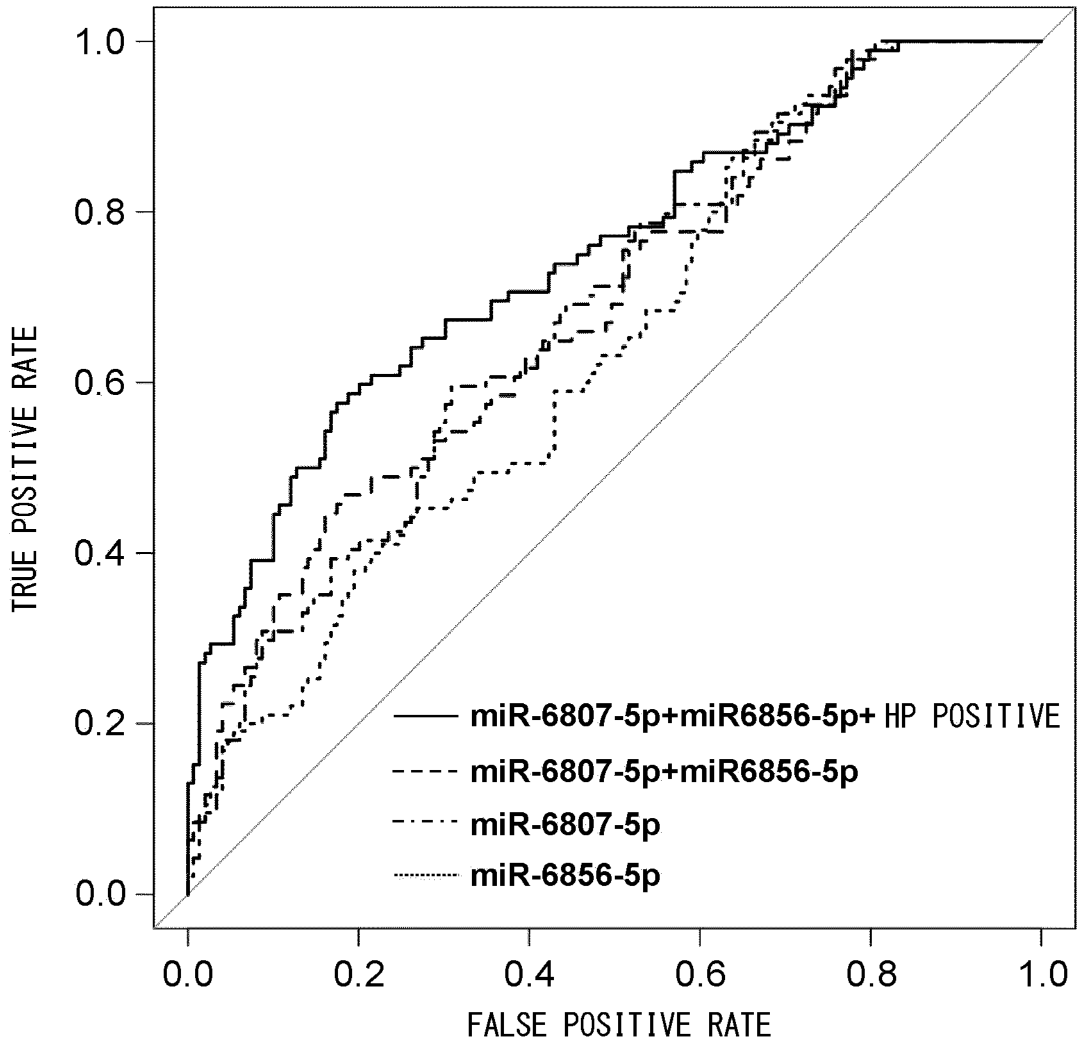


| BIOMARKER | AUC (95% CI) |
|---|---|
| **miR-6807-5p+miR-6856-5p+** HP POSITIVE | **0.748 (0.683-0.812)** |
| **miR-6807-5p+miR-6856-5p** | 0.684 (0.616-0.752) |
| **miR-6807-5p** | 0.683 (0.616-0.750) |
| **miR-6856-5p** | 0.640 (0.570-0.709) |

FIG. 21

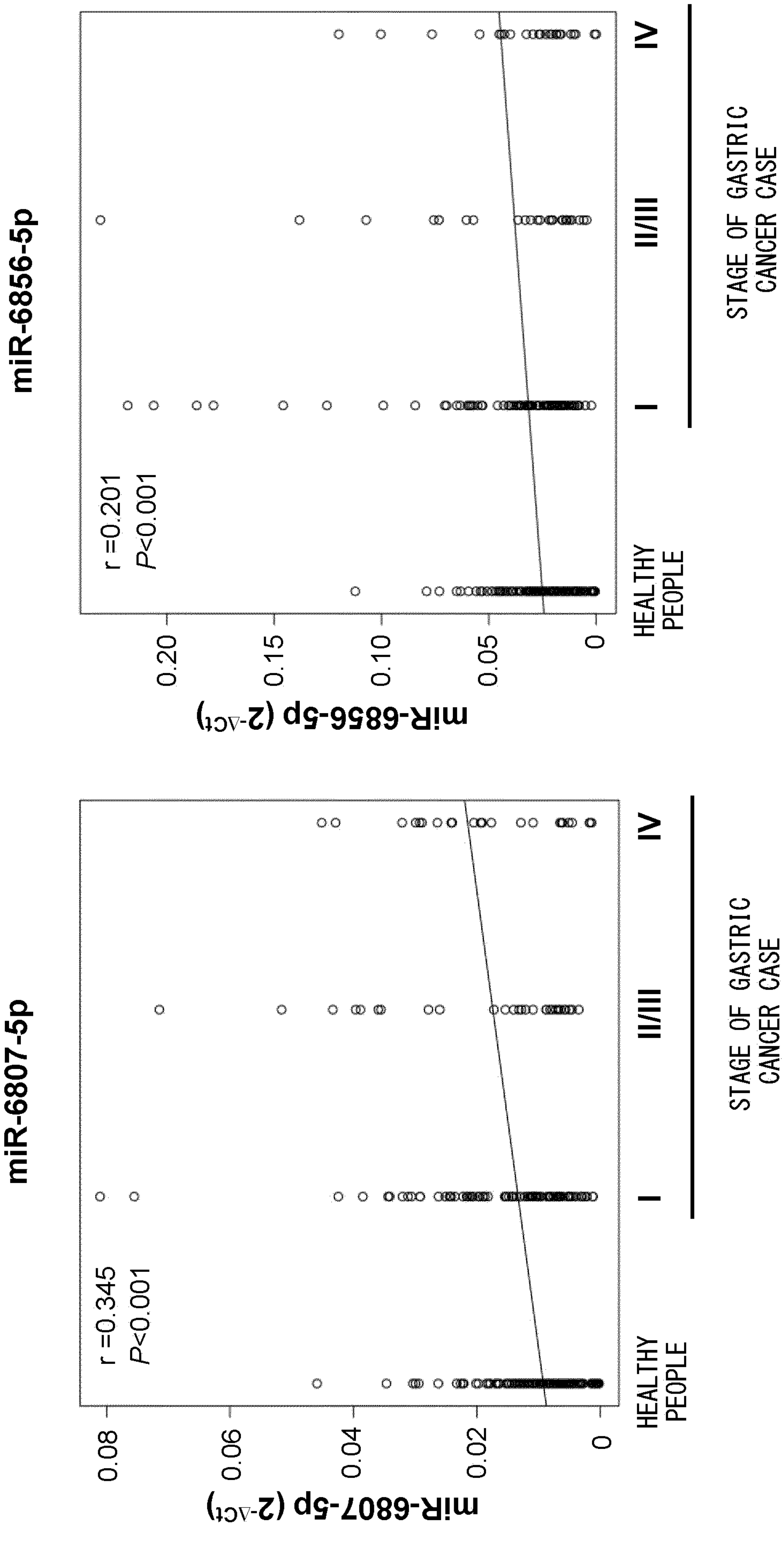
miR-6807-5p
r =0.345
P<0.001
miR-6807-5p (2-ΔCt)
0.08
0.06
0.04
0.02
0
HEALTHY PEOPLE
I
II/III
IV
STAGE OF GASTRIC CANCER CASE
miR-6856-5p
r =0.201
P<0.001
miR-6856-5p (2-ΔCt)
0.20
0.15
0.10
0.05
0
HEALTHY PEOPLE
I
II/III
IV
STAGE OF GASTRIC CANCER CASE

FIG. 22

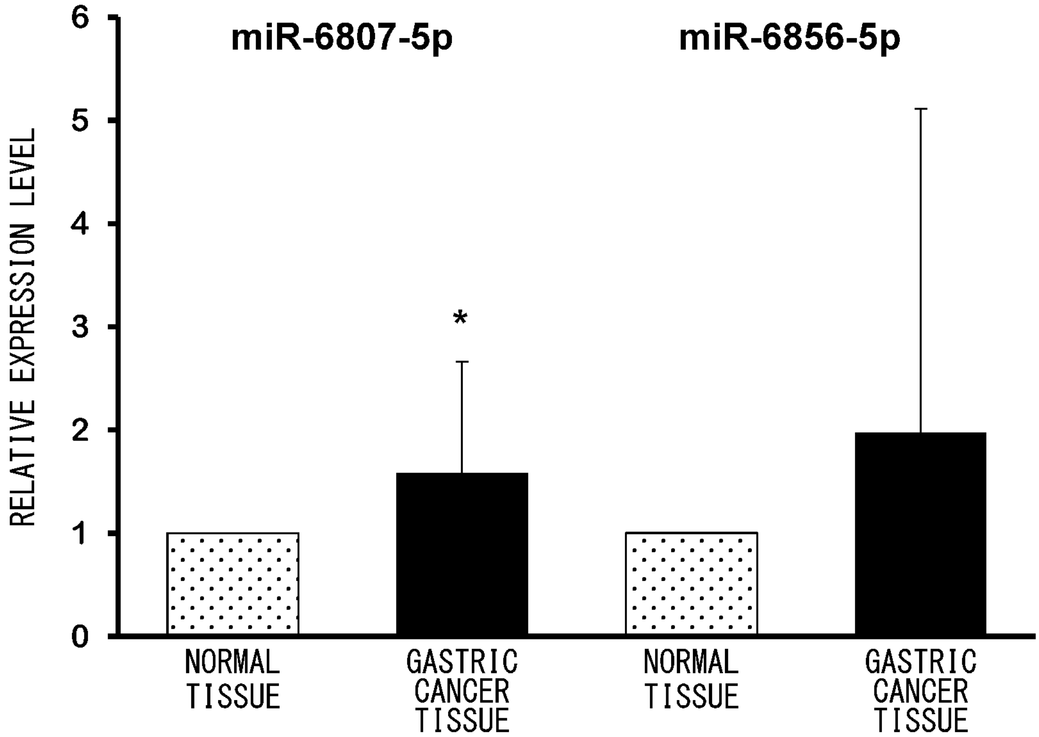


| | | $2^{-\Delta\Delta Ct}$ | p VALUE |
|---|---|---|---|
| NORMAL TISSUE **(n=20)** | | 1 | |
| GASTRIC CANCER TISSUE **(n=20)** | **miR-6807-5p** | 1.59 ± 1.08 | 0.025 |
| | **miR-6856-5p** | 1.97 ± 3.14 | 0.181 |

FIG. 23

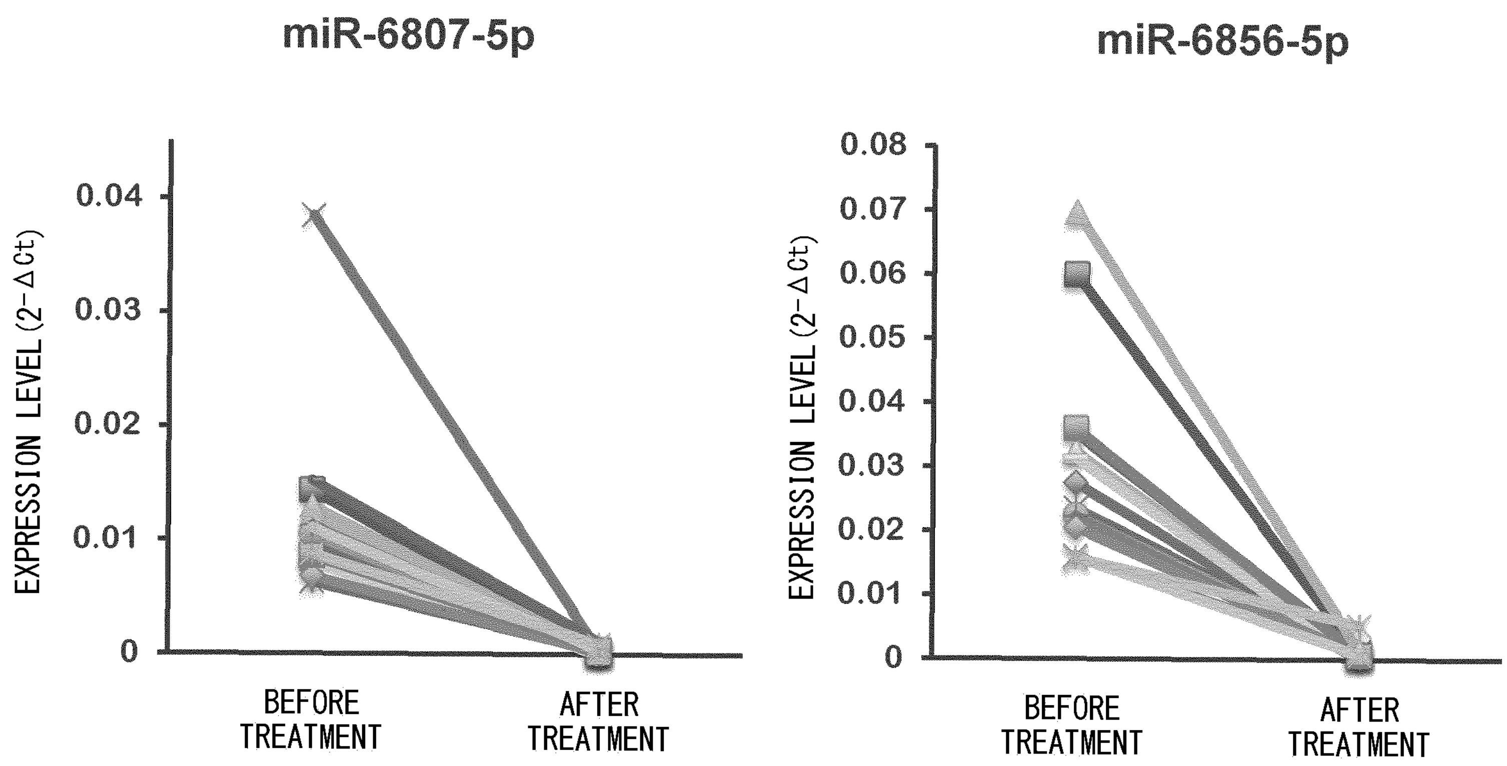


| (N=14) | BEFORE TREATMENT ($2^{-\Delta Ct}$) | AFTER TREATMENT ($2^{-\Delta Ct}$) | p VALUE |
|---|---|---|---|
| miR-6807-5p | 0.012±0.008 | 0.000±0.000 | <0.001 |
| miR-6856-5p | 0.031±0.016 | 0.000±0.000 | 0.149 |

## INTERNATIONAL SEARCH REPORT

International application No.
PCT/JP2019/021098

A. CLASSIFICATION OF SUBJECT MATTER
Int.Cl. C12N15/11(2006.01)i, C12Q1/6851(2018.01)i, C12Q1/6886(2018.01)i, G01N33/50(2006.01)i

According to International Patent Classification (IPC) or to both national classification and IPC

B. FIELDS SEARCHED

Minimum documentation searched (classification system followed by classification symbols)
Int.Cl. C12N15/11, C12Q1/6851, C12Q1/6886, G01N33/50

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | YU, Y. et al., "MicroRNA profiling of human gastric cancer", Molecular Medicine Reports, 2009, vol. 2, pp. 963-970, ISSN:1791-3004, in particular, abstract | 1-3,19,22-23<br>1-23 |
| Y | RAI, R. P. et al., "Elevated expression of miR-223 and miR-21 in Helicobacter pylori induced gastric cancer patients", 17th International Congress on Infectious Diseases/International Journal of Infectious Diseases, 2016, vol. 45 Suppl., p. 147, ISSN: 1201-9712, entire text | 1-23 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 August 2019 (08.08.2019) | 20 August 2019 (20.08.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/021098

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2017-195803 A (THE KITASATO INSTITUTE) 02 November 2017, claims 1-8, paragraph [0037], example 1 & WO 2017/188315 A1, claims 1-8, paragraph [0037], example 1 | 7-18 |
| Y | HUANG, S. et al., "Serum microRNA expression profile as a diagnostic panel for gastric cancer", Japanese Journal of Clinical Oncology, 2016, vol. 46, no. 9, pp. 811-818, ISSN:1465-3621, in particular, abstract, fig. 1-3, tables 3-4 | 1-23 |
| Y | ZHANG, C. et al., "Three-microRNA signature identified by bioinformatics analysis predicts prognosis of gastric cancer patients", World J. Gastroenterol., March 2018, vol. 24, no. 11, pp. 1206-1215, ISSN: 2219-2840, in particular, abstract | 1-23 |
| Y | KAO, H. -W. et al., "Urine miR-21-5p as a potential non-invasive biomarker for gastric cancer", Oncotarget, 2017, vol. 8, pp. 56389-56397, ISSN: 1949-2553, in particular, abstract | 1-23 |
| Y | miRBase rel.21に対応したSurePrint miRNA Microarrays rel.21, [retrieved on 08 August 2019], Agilent Technology, 2015, entire text, <https://www.chem-agilent.com/pdf/Low_5991-6250JAJP.pdf>, non-official translation ("SurePrint miRNA Microarrays rel.21 corresponding to miRBase rel.21") | 1-23 |
| P,A | LINK, A. et al., "MicroRNAs as non-invasive diagnostic biomarkers for gastric cancer: Current insights and future perspectives", World J. Gastroenterol., August 2018, vol. 24, no. 30, pp. 3313-3329, ISSN: 2219-2840, entire text | 1-23 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018118340 A
- JP 2016192909 A
- JP 2016103761 W
- JP 2015194627 W
- JP 2015194615 W
- JP 2015190586 W
- JP 2015190542 W
- JP 2015182781 W
- JP 3433929 B
- JP 3883476 B

### Non-patent literature cited in the description

- MicroRNA profiling of human gastric cancer. *Molecular Medicine Reports,* November 2009
- **TSUGUNORI NOTOMI et al.** *Nucleic Acids Research,* 2000, vol. 28 (12), e63
- **KENTARO NAGAMINE ; KEIKO WATANABE et al.** *Clinical Chemistry,* 2001, vol. 47 (9), 1742-1743

SEQUENCE LISTING

<110> NAGOYA CITY UNIVERSITY

<120> BIOMARKER FOR STOMACH CANCER AND USE THEREOF

<130> NC18003P

<150> JP P2018-118340
<151> 2018-06-21

<160> 13

<170> PatentIn version 3.5

<210> 1
<211> 19
<212> RNA
<213> Homo sapiens

<400> 1
gagccaguug gacaggagc 19

<210> 2
<211> 20
<212> RNA
<213> Homo sapiens

<400> 2
gaaucggaaa ggaggcgccg 20

<210> 3
<211> 22
<212> RNA
<213> Homo sapiens

<400> 3
aauucccuug uagauaaccc gg 22

<210> 4
<211> 22
<212> RNA
<213> Homo sapiens

<400> 4
caggaaggau uuagggacag gc 22

<210> 5
<211> 22
<212> RNA
<213> Homo sapiens

<400> 5
ugugucccggg aaguggagga gg 22

<210> 6
<211> 21
<212> RNA

<213> Homo sapiens

<400> 6
uacuuuucua gguuguuggg g 21

<210> 7
<211> 24
<212> RNA
<213> Homo sapiens

<400> 7
ucugggcaca ggcggaugga cagg 24

<210> 8
<211> 24
<212> RNA
<213> Homo sapiens

<400> 8
aggagcuauc cacuccaggu gucc 24

<210> 9
<211> 46
<212> RNA
<213> Homo sapiens

<400> 9
caggcagaag uggggcugac aggcaggcag aagugggcu gacagg 46

<210> 10
<211> 22
<212> RNA
<213> Homo sapiens

<400> 10
cugggggugg ggggcugggc gu 22

<210> 11
<211> 22
<212> RNA
<213> Homo sapiens

<400> 11
gugagccagu ggaauggaga gg 22

<210> 12
<211> 24
<212> RNA
<213> Homo sapiens

<400> 12
aagagaggag caguggugcu gugg 24

<210> 13
<211> 21
<212> RNA

<213> Homo sapiens

<400> 13
ugagggaccc aggacaggag a 21